# EUROPEAN PATENT APPLICATION

(11) **EP 4 803 626 A1**
(43) Date of publication of application: **09.09.2026**
(21) Application number: 24884909.3
(22) Date of filing: 31.10.2024
(51) Int. Cl.: C12N 15/113, A61K 31/713

(54) **DSRNA MOLECULE FOR INHIBITING EXPRESSION OF COMPLEMENT MASP2 GENE AND USE THEREOF**

(30) Priority: 31.10.2023 CN 202311434945
(71) Applicant: CSPC Zhongqi Pharmaceutical Technology (Shijiazhuang) Co., Ltd., Shijiazhuang, Hebei 050035 (CN)
(72) Inventor: ZHANG, Xueyan, Shijiazhuang, Hebei 050035 (CN); SHI, Yanjun, Shijiazhuang, Hebei 050035 (CN); WANG, Lingyu, Shijiazhuang, Hebei 050035 (CN); SU, Xiaoye, Shijiazhuang, Hebei 050035 (CN); ZHONG, Qiang, Shijiazhuang, Hebei 050035 (CN)
(74) Representative: Lavoix
(86) International application number: PCT/CN2024/128920
(87) International publication number: WO 2025/092910

(57) **Abstract**

Provided are a modified dsRNA molecule and a use thereof. Specifically provided is a dsRNA molecule for inhibiting the expression of the MASP2 gene, said molecule comprising a sense strand and an antisense strand that complement each other and form a double-stranded region, and the sense strand and/or the antisense strand comprising 15-25 nucleotides or being composed of 15-25 nucleotides. The described dsRNA molecule can be used to treat and/or prevent diseases mediated by the MASP2 gene.

## Description

### TECHNICAL FIELD

This application belongs to the field of molecular biology, and relates to a modified dsRNA molecule and use thereof, particularly to a dsRNA molecule for inhibiting expression of complement MASP2 gene and its pharmaceutical composition, as well as a method for reducing the expression level of complement MASP2 gene by using the dsRNA molecule or its pharmaceutical composition.

### BACKGROUND ART

RNA interference (RNAi) refers to a highly conserved phenomenon of efficient and specific degradation of homologous mRNA which is induced by double-stranded RNA (dsRNA) during evolution. RNAi is a common monitoring mechanism in eukaryotes that defends against viral invasion, inhibits transposon activity, and regulates gene expression. Small interfering RNA (dsRNA) is a class of short double-stranded RNA molecules with a length of 19-30 bp, and is one of the important tools in RNAi technology. In a natural organism, after dsRNA enters a cell, it is particularly recognized by the Dicer enzyme and cleaved into small RNA fragments with a length of 21-23 nucleotides (i.e., dsRNA). Then the resulting dsRNA fragments unwind into single strands, and form complexes with certain proteins (RISC). RISC can bind to mRNA that is complementary to dsRNA in the cell and cleave the mRNA, thereby causing it to be degraded, resulting in the inability to synthesize proteins and producing a gene "silencing" phenomenon. In industrial production, people tend to chemically synthesize dsRNA and modify it to further improve the stability and efficacy of dsRNA medicaments. In recent years, breakthroughs have been made in the research of dsRNA-based medicaments, and several dsRNA medicaments targeting rare diseases have been approved by the FDA. The therapeutic applications of dsRNA medicaments have gradually expanded from rare diseases to common diseases.

Overactivation or inhibition of the complement system plays an important role in the pathogenesis of a wide range of diseases, from acute inflammation such as eye disease and periodontal disease to chronic disease such as cancer, autoimmune disease, neurodegenerative disease, kidney disease, and chronic hemolytic disease. There are three known complement activation pathways: the classical pathway, the alternative pathway, and the lectin pathway. Complement protein MASP2 is the initiating molecule of the complement lectin pathway, which in turn triggers the complement cascade reaction. Overactivation of complement is involved in the development of various diseases, such as ophthalmic disease: age-related macular degeneration (AMD); CNS/PNS disease: Alzheimer's disease (AD), myasthenia gravis (gMG); kidney disease: atypical hemolytic uremic syndrome (aHUS), C3 glomerulonephropathy (C3G) and IgA nephropathy; and hematological disease: paroxysmal nocturnal hemoglobinuria (PNH), thrombotic microangiopathy (TMAs). Targeting the MASP2 protein can block the complement cascade signaling mediated by the lectin pathway. Up to now, several medicaments targeting complement have been used in clinical research, but they are mainly antibody medicaments. Currently, antibody medicaments targeting MADP-2 are still in the clinical research stage, but they have shown great therapeutic potential in patients with IgA nephropathy. Therefore, there is a need for replacement and combination therapies for patients with complement component MASP2-related diseases in some refractory disease areas.

### SUMMARY

This application provides a modified double-stranded RNAi molecule, pharmaceutical compositions comprising the modified double-stranded RNAi molecule, and use thereof.

Particularly, in one aspect, this application provides an engineered dsRNA molecule for inhibiting the expression of the complement MASP2 gene, wherein the molecule comprises a sense strand and an antisense strand complementary to each other to form a double-stranded region, the sense strand and/or the antisense strand comprises or consists of 15-25 nucleotides, the antisense strand is complementary to at least 15, 16, 17, 18, 19, 20 or 21 consecutive nucleotides of the nucleic acid sequence set forth as SEQ ID NO: 37, 39, 41, 43, 45, 47, 49, 51, 53, 55, 71 or 77, and the length of the double-stranded region is 15-25 bp.

In some embodiments, at least one nucleotide in the dsRNA molecule is modified, and the modification is any one or more selected from the group consisting of: locked nucleic acid modification, open-ring or unlocked nucleic acid modification, 2'-methoxyethyl modification, 2'-O-methyl modification, 2'-O-allyl modification, 2'-C-allyl modification, 2'-fluoro modification, 2'-deoxy modification, phosphorothioate backbone modification, DNA modification, and ligand modification.

In some embodiments, for its naked sequence: the sense strand of the dsRNA molecule comprises or consists of the nucleic acid sequence set forth as SEQ ID NO:37, and the antisense strand comprises or consists of the nucleic acid sequence set forth as SEQ ID NO:38; the sense strand of the dsRNA molecule comprises or consists of the nucleic acid sequence set forth as SEQ ID NO:39, and the antisense strand comprises or consists of the nucleic acid sequence set forth as SEQ ID NO:40; the sense strand of the dsRNA molecule comprises or consists of the nucleic acid sequence set forth as SEQ ID NO:41, and the antisense strand comprises or consists of the nucleic acid sequence set forth as SEQ ID NO:42; the sense strand of the dsRNA molecule comprises or consists of the nucleic acid sequence set forth as SEQ ID NO:43, and the antisense strand comprises or consists of the nucleic acid sequence set forth as SEQ ID NO:44; the sense strand of the dsRNA molecule comprises or consists of the nucleic acid sequence set forth as SEQ ID NO:45, and the antisense strand comprises or consists of the nucleic acid sequence set forth as SEQ ID NO:46; the sense strand of the dsRNA molecule comprises or consists of the nucleic acid sequence set forth as SEQ ID NO:47, and the antisense strand comprises or consists of the nucleic acid sequence set forth as SEQ ID NO:48; the sense strand of the dsRNA molecule comprises or consists of the nucleic acid sequence set forth as SEQ ID NO:49, and the antisense strand comprises or consists of the nucleic acid sequence set forth as SEQ ID NO:50; the sense strand of the dsRNA molecule comprises or consists of the nucleic acid sequence set forth as SEQ ID NO:51, and the antisense strand comprises or consists of the nucleic acid sequence set forth as SEQ ID NO:52; the sense strand of the dsRNA molecule comprises or consists of the nucleic acid sequence set forth as SEQ ID NO:53, and the antisense strand comprises or consists of the nucleic acid sequence set forth as SEQ ID NO:54; the sense strand of the dsRNA molecule comprises or consists of the nucleic acid sequence set forth as SEQ ID NO:55, and the antisense strand comprises or consists of the nucleic acid sequence set forth as SEQ ID NO:56; the sense strand of the dsRNA molecule comprises or consists of the nucleic acid sequence set forth as SEQ ID NO:71, and the antisense strand comprises or consists of the nucleic acid sequence set forth as SEQ ID NO:72; or the sense strand of the dsRNA molecule comprises or consists of the nucleic acid sequence set forth as SEQ ID NO:77, and the antisense strand comprises or consists of the nucleic acid sequence set forth as SEQ ID NO:78.

In some embodiments, the modification manner of the dsRNA molecule comprises: (1) a sense strand: 19-23 nt in length, such as 19, 20, 21, 22, or 23 nt; consisting of alternating 2'-O-methyl and 2'-fluoro modified regions, wherein the number of the consecutive nucleotides in each modified region is any one of 1, 2, 3, 4, 5, 6, 7, 8, 9, 10, 11, or 12 nucleotides; and (2) an antisense strand: 19-25 nt in length, such as 19, 20, 21, 22, 23nt, 24n or 25nt; consisting of alternating 2'-O-methyl modified region, 2'-fluoro modified region, unmodified region and/or DNA region, wherein the number of the consecutive nucleotides in each modified region is any one of 1, 2, 3, 4, 5, 6, 7, 8, 9, 10, 11 or 12 nucleotides; the modification manners of the first modified region from the 5' end and the 3' end are the same; furthermore, in the sense strand and antisense strand, the consecutive nucleotide regions at positions 1-2, 1-3, 1-4, 1-5, 1-6, or 1-7 starting from the 5' end, and optionally the consecutive nucleotide regions at positions 1-2, 1-3, 1-4, 1-5, 1-6, or 1-7 starting from the 3' end, are all linked by a phosphorothioate backbone; preferably, the consecutive nucleotide regions at positions 1-3 starting from the 5' end, and optionally the consecutive nucleotide regions at positions 1-3 starting from the 3' end, are all linked by a phosphorothioate backbone.

In some embodiments, the ligand in the ligand modification is any one or more selected from the group consisting of: cholesterol, biotin, vitamin, galactose derivative or analog, lactose derivative or analog, N-acetylgalactosamine derivative or analog, and N-acetylglucosamine derivative or analog.

In some embodiments, the ligand is linked to the 3' terminal nucleotide of the sense strand and/or antisense strand; the conjugation is linked to a base or a sugar ring; preferably, the ligand is linked to a sugar ring; more preferably, the ligand is linked to the 3' position of the sugar ring.

In some embodiments, the ligand is one or more GalNAc derivatives linked by a divalent or trivalent branched structure;
preferably, the GalNAc derivative comprises the following structure: 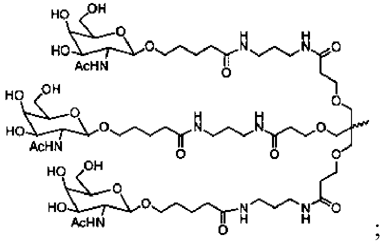
more preferably, the ligand is L96 with a structure shown in the following Formula I:

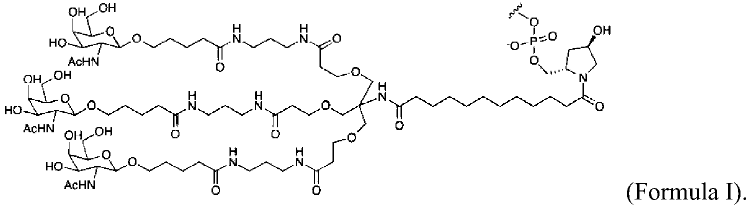

In some embodiments, the dsRNA molecule comprises modification motifs selected from any one of the following items: (1) a sense strand: NmsNmsNmNmNmNmNfNmNfNfNfNmNmNmNmNmNmNmNmsNmsNm, and an antisense strand: NmsNfsNmNmNmNmNmNmNmNmNmNmNmNfNmNfNmNmNmNmNmsNmsNm; (2) a sense strand: NmsNmsNmNmNmNmNfNmNfNfNfNmNmNmNmNmNmNmNmNmNm, and an antisense strand: NmsNfsNmNmNmNmNmNmNmNmNmNmNmNfNmNfNmNmNmNmNmsNmsNm; wherein Nm represents a ribonucleotide modified with 2'-O-methyl; Nf represents a ribonucleotide modified with 2'-fluoro; and (s) indicates that the two adjacent nucleotides are linked by a phosphorothioate backbone.

In some embodiments, the double-stranded RNAi molecule comprises one or more of the following items: (1) a sense strand consisting of a nucleotide sequence having at least 90%, preferably 95%, 96%, 97%, 98%, 99%, or 100% identity with the nucleotide sequence and corresponding modifications set forth as SEQ ID NO: 1, and an antisense strand consisting of a nucleotide sequence having at least 90%, preferably 95%, 96%, 97%, 98%, 99%, or 100% identity with the nucleotide sequence and corresponding modifications set forth as SEQ ID NO:2; (2) a sense strand consisting of a nucleotide sequence having at least 90%, preferably 95%, 96%, 97%, 98%, 99%, or 100% identity with the nucleotide sequence and corresponding modifications set forth as SEQ ID NO:3, and an antisense strand consisting of a nucleotide sequence having at least 90%, preferably 95%, 96%, 97%, 98%, 99%, or 100% identity with the nucleotide sequence and corresponding modifications set forth as SEQ ID NO:4; (3) a sense strand consisting of a nucleotide sequence having at least 90%, preferably 95%, 96%, 97%, 98%, 99%, or 100% identity with the nucleotide sequence and corresponding modifications set forth as SEQ ID NO:5, and an antisense strand consisting of a nucleotide sequence having at least 90%, preferably 95%, 96%, 97%, 98%, 99%, or 100% identity with the nucleotide sequence and corresponding modifications set forth as SEQ ID NO:6; (4) a sense strand consisting of a nucleotide sequence having at least 90%, preferably 95%, 96%, 97%, 98%, 99%, or 100% identity with the nucleotide sequence and corresponding modifications set forth as SEQ ID NO:7, and an antisense strand consisting of a nucleotide sequence having at least 90%, preferably 95%, 96%, 97%, 98%, 99%, or 100% identity with the nucleotide sequence and corresponding modifications set forth as SEQ ID NO:8; (5) a sense strand consisting of a nucleotide sequence having at least 90%, preferably 95%, 96%, 97%, 98%, 99%, or 100% identity with the nucleotide sequence and corresponding modifications set forth as SEQ ID NO:9, and an antisense strand consisting of a nucleotide sequence having at least 90%, preferably 95%, 96%, 97%, 98%, 99%, or 100% identity with the nucleotide sequence and corresponding modifications set forth as SEQ ID NO:10; (6) a sense strand consisting of a nucleotide sequence having at least 90%, preferably 95%, 96%, 97%, 98%, 99%, or 100% identity with the nucleotide sequence and corresponding modifications set forth as SEQ ID NO:11, and an antisense strand consisting of a nucleotide sequence having at least 90%, preferably 95%, 96%, 97%, 98%, 99%, or 100% identity with the nucleotide sequence and corresponding modifications set forth as SEQ ID NO:12; (7) a sense strand consisting of a nucleotide sequence having at least 90%, preferably 95%, 96%, 97%, 98%, 99%, or 100% identity with the nucleotide sequence and corresponding modifications set forth as SEQ ID NO:13, and an antisense strand consisting of a nucleotide sequence having at least 90%, preferably 95%, 96%, 97%, 98%, 99%, or 100% identity with the nucleotide sequence and corresponding modifications set forth as SEQ ID NO:14; (8) a sense strand consisting of a nucleotide sequence having at least 90%, preferably 95%, 96%, 97%, 98%, 99%, or 100% identity with the nucleotide sequence and corresponding modifications set forth as SEQ ID NO:15, and an antisense strand consisting of a nucleotide sequence having at least 90%, preferably 95%, 96%, 97%, 98%, 99%, or 100% identity with the nucleotide sequence and corresponding modifications set forth as SEQ ID NO:16; (9) a sense strand consisting of a nucleotide sequence having at least 90%, preferably 95%, 96%, 97%, 98%, 99%, or 100% identity with the nucleotide sequence and corresponding modifications set forth as SEQ ID NO:17, and an antisense strand consisting of a nucleotide sequence having at least 90%, preferably 95%, 96%, 97%, 98%, 99%, or 100% identity with the nucleotide sequence and corresponding modifications set forth as SEQ ID NO:18; (10) a sense strand consisting of a nucleotide sequence having at least 90%, preferably 95%, 96%, 97%, 98%, 99%, or 100% identity with the nucleotide sequence and corresponding modifications set forth as SEQ ID NO:19, and an antisense strand consisting of a nucleotide sequence having at least 90%, preferably 95%, 96%, 97%, 98%, 99%, or 100% identity with the nucleotide sequence and corresponding modifications set forth as SEQ ID NO:20; (11) a sense strand consisting of a nucleotide sequence having at least 90%, preferably 95%, 96%, 97%, 98%, 99%, or 100% identity with the nucleotide sequence and corresponding modifications set forth as SEQ ID NO:21, and an antisense strand consisting of a nucleotide sequence having at least 90%, preferably 95%, 96%, 97%, 98%, 99%, or 100% identity with the nucleotide sequence and corresponding modifications set forth as SEQ ID NO:22; (12) a sense strand consisting of a nucleotide sequence having at least 90%, preferably 95%, 96%, 97%, 98%, 99%, or 100% identity with the nucleotide sequence and corresponding modifications set forth as SEQ ID NO:23, and an antisense strand consisting of a nucleotide sequence having at least 90%, preferably 95%, 96%, 97%, 98%, 99%, or 100% identity with the nucleotide sequence and corresponding modifications set forth as SEQ ID NO:24; (13) a sense strand consisting of a nucleotide sequence having at least 90%, preferably 95%, 96%, 97%, 98%, 99%, or 100% identity with the nucleotide sequence and corresponding modifications set forth as SEQ ID NO:25, and an antisense strand consisting of a nucleotide sequence having at least 90%, preferably 95%, 96%, 97%, 98%, 99%, or 100% identity with the nucleotide sequence and corresponding modifications set forth as SEQ ID NO:2; (14) a sense strand consisting of a nucleotide sequence having at least 90%, preferably 95%, 96%, 97%, 98%, 99%, or 100% identity with the nucleotide sequence and corresponding modifications set forth as SEQ ID NO:26, and an antisense strand consisting of a nucleotide sequence having at least 90%, preferably 95%, 96%, 97%, 98%, 99%, or 100% identity with the nucleotide sequence and corresponding modifications set forth as SEQ ID NO:4; (15) a sense strand consisting of a nucleotide sequence having at least 90%, preferably 95%, 96%, 97%, 98%, 99%, or 100% identity with the nucleotide sequence and corresponding modifications set forth as SEQ ID NO:27, and an antisense strand consisting of a nucleotide sequence having at least 90%, preferably 95%, 96%, 97%, 98%, 99%, or 100% identity with the nucleotide sequence and corresponding modifications set forth as SEQ ID NO:6; (16) a sense strand consisting of a nucleotide sequence having at least 90%, preferably 95%, 96%, 97%, 98%, 99%, or 100% identity with the nucleotide sequence and corresponding modifications set forth as SEQ ID NO:28, and an antisense strand consisting of a nucleotide sequence having at least 90%, preferably 95%, 96%, 97%, 98%, 99%, or 100% identity with the nucleotide sequence and corresponding modifications set forth as SEQ ID NO:8; (17) a sense strand consisting of a nucleotide sequence having at least 90%, preferably 95%, 96%, 97%, 98%, 99%, or 100% identity with the nucleotide sequence and corresponding modifications set forth as SEQ ID NO:29, and an antisense strand consisting of a nucleotide sequence having at least 90%, preferably 95%, 96%, 97%, 98%, 99%, or 100% identity with the nucleotide sequence and corresponding modifications set forth as SEQ ID NO:10; (18) a sense strand consisting of a nucleotide sequence having at least 90%, preferably 95%, 96%, 97%, 98%, 99%, or 100% identity with the nucleotide sequence and corresponding modifications set forth as SEQ ID NO:30, and an antisense strand consisting of a nucleotide sequence having at least 90%, preferably 95%, 96%, 97%, 98%, 99%, or 100% identity with the nucleotide sequence and corresponding modifications set forth as SEQ ID NO:12; (19) a sense strand consisting of a nucleotide sequence having at least 90%, preferably 95%, 96%, 97%, 98%, 99%, or 100% identity with the nucleotide sequence and corresponding modifications set forth as SEQ ID NO:31, and an antisense strand consisting of a nucleotide sequence having at least 90%, preferably 95%, 96%, 97%, 98%, 99%, or 100% identity with the nucleotide sequence and corresponding modifications set forth as SEQ ID NO:14; (20) a sense strand consisting of a nucleotide sequence having at least 90%, preferably 95%, 96%, 97%, 98%, 99%, or 100% identity with the nucleotide sequence and corresponding modifications set forth as SEQ ID NO:32, and an antisense strand consisting of a nucleotide sequence having at least 90%, preferably 95%, 96%, 97%, 98%, 99%, or 100% identity with the nucleotide sequence and corresponding modifications set forth as SEQ ID NO:16; (21) a sense strand consisting of a nucleotide sequence having at least 90%, preferably 95%, 96%, 97%, 98%, 99%, or 100% identity with the nucleotide sequence and corresponding modifications set forth as SEQ ID NO:33, and an antisense strand consisting of a nucleotide sequence having at least 90%, preferably 95%, 96%, 97%, 98%, 99%, or 100% identity with the nucleotide sequence and corresponding modifications set forth as SEQ ID NO:18; (22) a sense strand consisting of a nucleotide sequence having at least 90%, preferably 95%, 96%, 97%, 98%, 99%, or 100% identity with the nucleotide sequence and corresponding modifications set forth as SEQ ID NO:34, and an antisense strand consisting of a nucleotide sequence having at least 90%, preferably 95%, 96%, 97%, 98%, 99%, or 100% identity with the nucleotide sequence and corresponding modifications set forth as SEQ ID NO:20; (23) a sense strand consisting of a nucleotide sequence having at least 90%, preferably 95%, 96%, 97%, 98%, 99%, or 100% identity with the nucleotide sequence and corresponding modifications set forth as SEQ ID NO:35, and an antisense strand consisting of a nucleotide sequence having at least 90%, preferably 95%, 96%, 97%, 98%, 99%, or 100% identity with the nucleotide sequence and corresponding modifications set forth as SEQ ID NO:22; and (24) a sense strand consisting of a nucleotide sequence having at least 90%, preferably 95%, 96%, 97%, 98%, 99%, or 100% identity with the nucleotide sequence and corresponding modifications set forth as SEQ ID NO:36, and an antisense strand consisting of a nucleotide sequence having at least 90%, preferably 95%, 96%, 97%, 98%, 99%, or 100% identity with the nucleotide sequence and corresponding modifications set forth as SEQ ID NO:24.

In some embodiments, the nucleic acid sequences of the double-stranded RNAi molecule comprise one or more of the following items: (1) a sense strand with a nucleotide sequence consisting of SEQ ID NO:1 and 0-5 additional nucleotides at the 5' and/or 3' ends, and an antisense strand with the nucleotide sequence consisting of SEQ ID NO:2 and 0-5 additional nucleotides at the 5' and/or 3' ends; (2) a sense strand with a nucleotide sequence consisting of SEQ ID NO:3 and 0-5 additional nucleotides at the 5' and/or 3' ends, and an antisense strand with the nucleotide sequence consisting of SEQ ID NO:4 and 0-5 additional nucleotides at the 5' and/or 3' ends; (3) a sense strand with a nucleotide sequence consisting of SEQ ID NO:5 and 0-5 additional nucleotides at the 5' and/or 3' ends, and an antisense strand with the nucleotide sequence consisting of SEQ ID NO:6 and 0-5 additional nucleotides at the 5' and/or 3' ends; (4) a sense strand with a nucleotide sequence consisting of SEQ ID NO:7 and 0-5 additional nucleotides at the 5' and/or 3' ends, and an antisense strand with the nucleotide sequence consisting of SEQ ID NO:8 and 0-5 additional nucleotides at the 5' and/or 3' ends; (5) a sense strand with a nucleotide sequence consisting of SEQ ID NO:9 and 0-5 additional nucleotides at the 5' and/or 3' ends, and an antisense strand with the nucleotide sequence consisting of SEQ ID NO:10 and 0-5 additional nucleotides at the 5' and/or 3' ends; (6) a sense strand with a nucleotide sequence consisting of SEQ ID NO:11 and 0-5 additional nucleotides at the 5' and/or 3' ends, and an antisense strand with the nucleotide sequence consisting of SEQ ID NO:12 and 0-5 additional nucleotides at the 5' and/or 3' ends; (7) a sense strand with a nucleotide sequence consisting of SEQ ID NO:13 and 0-5 additional nucleotides at the 5' and/or 3' ends, and an antisense strand with the nucleotide sequence consisting of SEQ ID NO:14 and 0-5 additional nucleotides at the 5' and/or 3' ends; (8) a sense strand with a nucleotide sequence consisting of SEQ ID NO:15 and 0-5 additional nucleotides at the 5' and/or 3' ends, and an antisense strand with the nucleotide sequence consisting of SEQ ID NO:16 and 0-5 additional nucleotides at the 5' and/or 3' ends; (9) a sense strand with a nucleotide sequence consisting of SEQ ID NO:17 and 0-5 additional nucleotides at the 5' and/or 3' ends, and an antisense strand with the nucleotide sequence consisting of SEQ ID NO:18 and 0-5 additional nucleotides at the 5' and/or 3' ends; (10) a sense strand with a nucleotide sequence consisting of SEQ ID NO:19 and 0-5 additional nucleotides at the 5' and/or 3' ends, and an antisense strand with the nucleotide sequence consisting of SEQ ID NO:20 and 0-5 additional nucleotides at the 5' and/or 3' ends; (11) a sense strand with a nucleotide sequence consisting of SEQ ID NO:21 and 0-5 additional nucleotides at the 5' and/or 3' ends, and an antisense strand with the nucleotide sequence consisting of SEQ ID NO:22 and 0-5 additional nucleotides at the 5' and/or 3' ends; (12) a sense strand with a nucleotide sequence consisting of SEQ ID NO:23 and 0-5 additional nucleotides at the 5' and/or 3' ends, and an antisense strand with the nucleotide sequence consisting of SEQ ID NO:24 and 0-5 additional nucleotides at the 5' and/or 3' ends; (13) a sense strand with a nucleotide sequence consisting of SEQ ID NO:25 and 0-5 additional nucleotides at the 5' and/or 3' ends, and an antisense strand with the nucleotide sequence consisting of SEQ ID NO:2 and 0-5 additional nucleotides at the 5' and/or 3' ends; (14) a sense strand with a nucleotide sequence consisting of SEQ ID NO:26 and 0-5 additional nucleotides at the 5' and/or 3' ends, and an antisense strand with the nucleotide sequence consisting of SEQ ID NO:4 and 0-5 additional nucleotides at the 5' and/or 3' ends; (15) a sense strand with a nucleotide sequence consisting of SEQ ID NO:27 and 0-5 additional nucleotides at the 5' and/or 3' ends, and an antisense strand with the nucleotide sequence consisting of SEQ ID NO:6 and 0-5 additional nucleotides at the 5' and/or 3' ends; (16) a sense strand with a nucleotide sequence consisting of SEQ ID NO:28 and 0-5 additional nucleotides at the 5' and/or 3' ends, and an antisense strand with the nucleotide sequence consisting of SEQ ID NO:8 and 0-5 additional nucleotides at the 5' and/or 3' ends; (17) a sense strand with a nucleotide sequence consisting of SEQ ID NO:29 and 0-5 additional nucleotides at the 5' and/or 3' ends, and an antisense strand with the nucleotide sequence consisting of SEQ ID NO:10 and 0-5 additional nucleotides at the 5' and/or 3' ends; (18) a sense strand with a nucleotide sequence consisting of SEQ ID NO:30 and 0-5 additional nucleotides at the 5' and/or 3' ends, and an antisense strand with the nucleotide sequence consisting of SEQ ID NO:12 and 0-5 additional nucleotides at the 5' and/or 3' ends; (19) a sense strand with a nucleotide sequence consisting of SEQ ID NO:31 and 0-5 additional nucleotides at the 5' and/or 3' ends, and an antisense strand with the nucleotide sequence consisting of SEQ ID NO:14 and 0-5 additional nucleotides at the 5' and/or 3' ends; (20) a sense strand with a nucleotide sequence consisting of SEQ ID NO:32 and 0-5 additional nucleotides at the 5' and/or 3' ends, and an antisense strand with the nucleotide sequence consisting of SEQ ID NO:16 and 0-5 additional nucleotides at the 5' and/or 3' ends; (21) a sense strand with a nucleotide sequence consisting of SEQ ID NO:33 and 0-5 additional nucleotides at the 5' and/or 3' ends, and an antisense strand with the nucleotide sequence consisting of SEQ ID NO:18 and 0-5 additional nucleotides at the 5' and/or 3' ends; (22) a sense strand with a nucleotide sequence consisting of SEQ ID NO:34 and 0-5 additional nucleotides at the 5' and/or 3' ends, and an antisense strand with the nucleotide sequence consisting of SEQ ID NO:20 and 0-5 additional nucleotides at the 5' and/or 3' ends; (23) a sense strand with a nucleotide sequence consisting of SEQ ID NO:35 and 0-5 additional nucleotides at the 5' and/or 3' ends, and an antisense strand with the nucleotide sequence consisting of SEQ ID NO:22 and 0-5 additional nucleotides at the 5' and/or 3' ends; and (24) a sense strand with a nucleotide sequence consisting of SEQ ID NO:36 and 0-5 additional nucleotides at the 5' and/or 3' ends, and an antisense strand with the nucleotide sequence consisting of SEQ ID NO:24 and 0-5 additional nucleotides at the 5' and/or 3' ends.

In some embodiments, the double-stranded RNAi molecule is selected from one or more of the following items: (1) a sense strand with the nucleotide sequence set forth as SEQ ID NO:25, and an antisense strand with the nucleotide sequence set forth as SEQ ID NO:2; (2) a sense strand with the nucleotide sequence set forth as SEQ ID NO:26, and an antisense strand with the nucleotide sequence set forth as SEQ ID NO:4; (3) a sense strand with the nucleotide sequence set forth as SEQ ID NO:27, and an antisense strand with the nucleotide sequence set forth as SEQ ID NO:6; (4) a sense strand with the nucleotide sequence set forth as SEQ ID NO:28, and an antisense strand with the nucleotide sequence set forth as SEQ ID NO:8; (5) a sense strand with the nucleotide sequence set forth as SEQ ID NO:29, and an antisense strand with the nucleotide sequence set forth as SEQ ID NO:10; (6) a sense strand with the nucleotide sequence set forth as SEQ ID NO:30, and an antisense strand with the nucleotide sequence set forth as SEQ ID NO:12; (7) a sense strand with the nucleotide sequence set forth as SEQ ID NO:31, and an antisense strand with the nucleotide sequence set forth as SEQ ID NO: 14; (8) a sense strand with the nucleotide sequence set forth as SEQ ID NO:32, and an antisense strand with the nucleotide sequence set forth as SEQ ID NO:16; (9) a sense strand with the nucleotide sequence set forth as SEQ ID NO:33, and an antisense strand with the nucleotide sequence set forth as SEQ ID NO: 18; (10) a sense strand with the nucleotide sequence set forth as SEQ ID NO:34, and an antisense strand with the nucleotide sequence set forth as SEQ ID NO:20; (11) a sense strand with the nucleotide sequence set forth as SEQ ID NO:35, and an antisense strand with the nucleotide sequence set forth as SEQ ID NO:22; and (12) a sense strand with the nucleotide sequence set forth as SEQ ID NO:36, and an antisense strand with the nucleotide sequence set forth as SEQ ID NO:24; wherein the 3'-OH at the 3' end of the sense strand is linked with an L96 ligand as shown in the following Formula I:

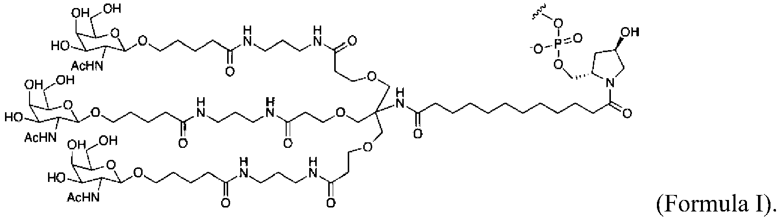

In another aspect, this application provides a biomaterial which is any one selected from the group consisting of:
(A) a DNA molecule capable of producing any one of the above double-stranded RNAi molecules;
(B) a vector capable of expressing any one of the above double-stranded RNAi molecules;
(C) a reagent or kit comprising any one of the above double-stranded RNAi molecules or the DNA molecule or vector described in (A) or (B) above;
(D) a pharmaceutical composition comprising any one of the above double-stranded RNAi molecules and other pharmaceutically acceptable components.

In another aspect, this application provides use of the dsRNA or double-stranded RNAi molecule, which is any one selected from the group consisting of:
(I) use of the dsRNA according to any one of claims 1-11 or the biomaterial according to claim 12 in inhibiting complement MASP2 gene expression or in the preparation of a product for inhibiting complement MASP2 gene expression;
(II) use of the dsRNA according to any one of claims 1-11 or the biomaterial according to claim 12 in the preparation of a product for reducing MASP2 proteins in serum;
(III) use of the dsRNA according to any one of claims 1-11 or the biomaterial according to claim 12 in the prevention and/or treatment of a disease mediated by the complement MASP2 gene, or in the preparation of a product for the prevention and/or treatment of a disease mediated by the complement MASP2 gene;
(IV) use of the dsRNA according to any one of claims 1-11 or the biomaterial according to claim 12 in alleviating symptoms of a disease mediated by the complement MASP2 gene, or in the preparation of a product for alleviating symptoms of a disease mediated by the complement MASP2 gene;
the disease mediated by the complement MASP2 gene comprises: ophthalmic disease, hematological disease, cardiovascular disease, autoimmune disease, kidney disease, neurological disease, or oncological disease; wherein the ophthalmic disease comprises dry/wet age-related macular degeneration (AMD), geographic atrophy (GA), etc.; the neurological disease comprises: Alzheimer's disease (AD), myasthenia gravis (gMG), etc.; the kidney disease comprises: typical hemolytic uremic syndrome (aHUS), C3 glomerulonephropathy (C3G), and IgA nephropathy; the hematological disease comprises: paroxysmal nocturnal hemoglobinuria (PNH), thrombotic microangiopathy (TMAs); the autoimmune disease comprises: rheumatoid arthritis, lupus erythematosus, etc.; and the oncological disease comprises: complement-related liver cancer or lung cancer.

It should be understood that, the aspects and embodiments of this application described herein comprise aspects and embodiments including "comprising", "consisting of", and "substantially consisting of." The preferred embodiments of this application have been described in detail above; however, this application is not limited thereto. Within the scope of the technical concept of this application, various simple modifications can be made to the technical solution of this application, including combining the various technical features in any other suitable manner. These simple modifications and combinations should also be regarded as the content disclosed herein and are all within the protection scope of this application.

### Technical effects:

The unexpected technical effects of this application are reflected in the following aspects: 1) the modified dsRNA molecules have high stability and high inhibitory activity; 2) while maintaining high inhibitory activity and stability, the ligand-modified dsRNA molecules also have good liver-targeting and the ability to promote cell endocytosis, thereby reducing the impact on other tissues or organs and reducing the amount of dsRNA molecules used, and thus achieving the purpose of reducing toxicity and reducing costs; 3) the ligand-modified dsRNA molecules can enter target cells and target tissues without transfection reagents, thereby reducing the negative effects of transfection reagents, such as cell or tissue toxicity. This provides a possibility for targeted therapy. While many modifications can be attempted to improve the performance of dsRNA, these attempts are often difficult to explain both the ability to mediate RNA interference and the enhanced stability in serum (e.g., increased resistance to nucleases and/or prolonged duration). The modified dsRNA of this application exhibits high stability while maintaining high inhibitory activity, thereby achieving unexpected technical effects.

### BRIEF DESCRIPTION OF THE DRAWINGS

Fig. 1 shows the results of high-throughput screening for dsRNA.
Fig. 2 shows the results of a single screening of modified candidate dsRNA sequences at 1 nM in HepG2 cells.
Fig. 3 shows the results of a single screening of modified candidate dsRNA sequences at 0.1 nM in HepG2 cells.

### DETAILED DESCRIPTION

This application provides a dsRNA molecule, reagent, kit, and pharmaceutical composition thereof for inhibiting the expression of complement MASP2 gene, as well as methods and uses of the above dsRNA molecule, reagent, kit, or pharmaceutical composition for inhibiting or reducing the expression of complement MASP2 gene, preventing or treating diseases or symptoms mediated by the complement MASP2 gene. The dsRNA molecule promotes sequence-specific degradation of MASP2 mRNA through RNAi, thereby inhibiting or reducing the expression level of complement MASP2 gene.

In one aspect, this application provides a double-stranded ribonucleic acid (dsRNA) molecule for inhibiting the expression of complement component MASP2, wherein the dsRNA comprises a sense strand and an antisense strand complementary to each other to form a double-stranded region, wherein the sense strand and/or antisense strand comprises or consists of 15-25 nucleotides, the antisense strand is complementary to at least 15, 16, 17, 18, 19, 20, or 21 consecutive nucleotides of the nucleic acid sequence set forth as SEQ ID NO: 37, 39, 41, 43, 45, 47, 49, 51, 53, 55, 71 or 77, and the length of the double-stranded region is 15-25 bp, preferably 19-21 bp.

In some embodiments, at least one nucleotide in the dsRNA molecule is modified, wherein the modification is any one or more selected from the group consisting of: locked nucleic acid (LNA) modification, open-ring or unlocked nucleic acid (UNA) modification, 2'-methoxyethyl modification, 2'-O-methyl modification, 2'-O-allyl modification, 2'-C-allyl modification, 2'-fluoro modification, 2'-deoxy modification, 2'-hydroxyl modification, phosphorothioate backbone modification, DNA modification, fluorescent probe modification, and ligand modification.

In some embodiments, the dsRNA molecule may be selected from the dsRNAs listed in Table 1 of Example 1; the dsRNA molecule may be selected from the dsRNAs having 80%, 85%, 90%, 91%, 92%, 93%, 94%, 95%, 96%, 97%, 98%, or 99% sequence identity with the dsRNAs listed in Table 1 of Example 1.

In some embodiments, the naked sequence of the dsRNA molecule may be any one or more selected from the group consisting of: the sense strand of the dsRNA molecule comprises or consists of the nucleic acid sequence set forth as SEQ ID NO:37, and the antisense strand comprises or consists of the nucleic acid sequence set forth as SEQ ID NO:38; the sense strand of the dsRNA molecule comprises or consists of the nucleic acid sequence set forth as SEQ ID NO:39, and the antisense strand comprises or consists of the nucleic acid sequence set forth as SEQ ID NO:40; the sense strand of the dsRNA molecule comprises or consists of the nucleic acid sequence set forth as SEQ ID NO:41, and the antisense strand comprises or consists of the nucleic acid sequence set forth as SEQ ID NO:42; the sense strand of the dsRNA molecule comprises or consists of the nucleic acid sequence set forth as SEQ ID NO:43, and the antisense strand comprises or consists of the nucleic acid sequence set forth as SEQ ID NO:44; the sense strand of the dsRNA molecule comprises or consists of the nucleic acid sequence set forth as SEQ ID NO:45, and the antisense strand comprises or consists of the nucleic acid sequence set forth as SEQ ID NO:46; the sense strand of the dsRNA molecule comprises or consists of the nucleic acid sequence set forth as SEQ ID NO:47, and the antisense strand comprises or consists of the nucleic acid sequence set forth as SEQ ID NO:48; the sense strand of the dsRNA molecule comprises or consists of the nucleic acid sequence set forth as SEQ ID NO:49, and the antisense strand comprises or consists of the nucleic acid sequence set forth as SEQ ID NO:50; the sense strand of the dsRNA molecule comprises or consists of the nucleic acid sequence set forth as SEQ ID NO:51, and the antisense strand comprises or consists of the nucleic acid sequence set forth as SEQ ID NO:52; the sense strand of the dsRNA molecule comprises or consists of the nucleic acid sequence set forth as SEQ ID NO:53, and the antisense strand comprises or consists of the nucleic acid sequence set forth as SEQ ID NO:54; the sense strand of the dsRNA molecule comprises or consists of the nucleic acid sequence set forth as SEQ ID NO:55, and the antisense strand comprises or consists of the nucleic acid sequence set forth as SEQ ID NO:56; the sense strand of the dsRNA molecule comprises or consists of the nucleic acid sequence set forth as SEQ ID NO:71, and the antisense strand comprises or consists of the nucleic acid sequence set forth as SEQ ID NO:72; or the sense strand of the dsRNA molecule comprises or consists of the nucleic acid sequence set forth as SEQ ID NO:77, and the antisense strand comprises or consists of the nucleic acid sequence set forth as SEQ ID NO:78.

In some embodiments, the modification manners of the dsRNA molecules provided in this application comprise: (1) a sense strand: 19-23 nt in length, such as 19, 20, 21, 22, or 23 nt, preferably 21 nt; consisting of alternating 2'-O-methyl and 2'-fluoro modified regions, wherein the number of the consecutive nucleotides in each modified region is any one of 1, 2, 3, 4, 5, 6, 7, 8, 9, 10, 11, or 12 nucleotides; and (2) an antisense strand: 19-25 nt in length, such as 19, 20, 21, 22, 23nt, 24n or 25nt, preferably 23 nt; consisting of alternating 2'-O-methyl modified region, 2'-fluoro modified region, unmodified region and/or DNA region, wherein the number of the consecutive nucleotides in each modified region is any one of 1, 2, 3, 4, 5, 6, 7, 8, 9, 10, 11 or 12 nucleotides; the modification manners of the first modified region from the 5' end and the 3' end are the same; furthermore, in the sense strand and antisense strand, the consecutive nucleotide regions at positions 1-2, 1-3, 1-4, 1-5, 1-6, or 1-7 starting from the 5' end, and optionally the consecutive nucleotide regions at positions 1-2, 1-3, 1-4, 1-5, 1-6, or 1-7 starting from the 3' end, are all linked by a phosphorothioate backbone; preferably, the consecutive nucleotide regions at positions 1-3 starting from the 5' end, and optionally the consecutive nucleotide regions at positions 1-3 starting from the 3' end, are all linked by a phosphorothioate backbone.

In some embodiments, the ligand in the ligand modification of the dsRNA molecule is any one or more selected from the group consisting of: cholesterol, biotin, vitamin, galactose derivative or analog, lactose derivative or analog, N-acetylgalactosamine derivative or analog, and N-acetylglucosamine derivative or analog.

In some embodiments, the ligand is linked to the 3' terminal nucleotide of the sense strand and/or antisense strand; the conjugation is linked to a base or a sugar ring; preferably, the ligand is linked to a sugar ring; more preferably, the ligand is linked to the 3' position of the sugar ring.

In some embodiments, the ligand in the ligand modification of the dsRNA molecule is one or more GalNAc derivatives linked by a divalent or trivalent branched structure;
preferably, the GalNAc derivative comprises the following structure: 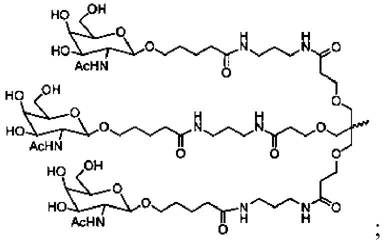
more preferably, the ligand is L96 with a structure shown in the following Formula I:

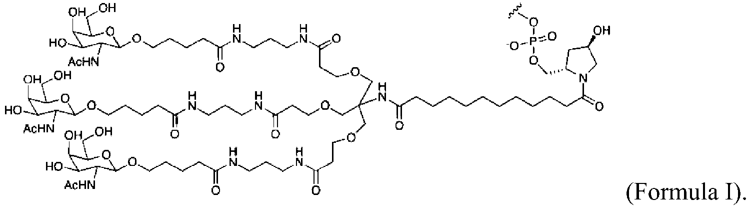

In some preferred embodiments, the dsRNA molecule comprises modification motifs selected from any one of the following items: (1) a sense strand: NmsNmsNmNmNmNmNfNmNfNfNfNmNmNmNmNmNmNmNmsNmsNm, and an antisense strand: NmsNfsNmNmNmNmNmNmNmNmNmNmNmNfNmNfNmNmNmNmNmsNmsNm; (2) a sense strand: NmsNmsNmNmNmNmNfNmNfNfNfNmNmNmNmNmNmNmNmNmNm, and an antisense strand: NmsNfsNmNmNmNmNmNmNmNmNmNmNmNfNmNfNmNmNmNmNmsNmsNm; wherein Nm represents a ribonucleotide modified with 2'-O-methyl; Nf represents a ribonucleotide modified with 2'-fluoro; and (s) indicates that the two adjacent nucleotides are linked by a phosphorothioate backbone.

In some preferred embodiments, the dsRNA molecule comprises one or more of the following items: (1) a sense strand consisting of a nucleotide sequence having at least 90%, preferably 95%, 96%, 97%, 98%, 99%, or 100% identity with the nucleotide sequence and corresponding modifications set forth as SEQ ID NO: 1, and an antisense strand consisting of a nucleotide sequence having at least 90%, preferably 95%, 96%, 97%, 98%, 99%, or 100% identity with the nucleotide sequence and corresponding modifications set forth as SEQ ID NO: 2; (2) a sense strand consisting of a nucleotide sequence having at least 90%, preferably 95%, 96%, 97%, 98%, 99%, or 100% identity with the nucleotide sequence and corresponding modifications set forth as SEQ ID NO: 3, and an antisense strand consisting of a nucleotide sequence having at least 90%, preferably 95%, 96%, 97%, 98%, 99%, or 100% identity with the nucleotide sequence and corresponding modifications set forth as SEQ ID NO: 4; (3) a sense strand consisting of a nucleotide sequence having at least 90%, preferably 95%, 96%, 97%, 98%, 99%, or 100% identity with the nucleotide sequence and corresponding modifications set forth as SEQ ID NO: 5, and an antisense strand consisting of a nucleotide sequence having at least 90%, preferably 95%, 96%, 97%, 98%, 99%, or 100% identity with the nucleotide sequence and corresponding modifications set forth as SEQ ID NO: 6; (4) a sense strand consisting of a nucleotide sequence having at least 90%, preferably 95%, 96%, 97%, 98%, 99%, or 100% identity with the nucleotide sequence and corresponding modifications set forth as SEQ ID NO: 7, and an antisense strand consisting of a nucleotide sequence having at least 90%, preferably 95%, 96%, 97%, 98%, 99%, or 100% identity with the nucleotide sequence and corresponding modifications set forth as SEQ ID NO: 8; (5) a sense strand consisting of a nucleotide sequence having at least 90%, preferably 95%, 96%, 97%, 98%, 99%, or 100% identity with the nucleotide sequence and corresponding modifications set forth as SEQ ID NO: 9, and an antisense strand consisting of a nucleotide sequence having at least 90%, preferably 95%, 96%, 97%, 98%, 99%, or 100% identity with the nucleotide sequence and corresponding modifications set forth as SEQ ID NO: 10; (6) a sense strand consisting of a nucleotide sequence having at least 90%, preferably 95%, 96%, 97%, 98%, 99%, or 100% identity with the nucleotide sequence and corresponding modifications set forth as SEQ ID NO: 11, and an antisense strand consisting of a nucleotide sequence having at least 90%, preferably 95%, 96%, 97%, 98%, 99%, or 100% identity with the nucleotide sequence and corresponding modifications set forth as SEQ ID NO: 12; (7) a sense strand consisting of a nucleotide sequence having at least 90%, preferably 95%, 96%, 97%, 98%, 99%, or 100% identity with the nucleotide sequence and corresponding modifications set forth as SEQ ID NO: 13, and an antisense strand consisting of a nucleotide sequence having at least 90%, preferably 95%, 96%, 97%, 98%, 99%, or 100% identity with the nucleotide sequence and corresponding modifications set forth as SEQ ID NO: 14; (8) a sense strand consisting of a nucleotide sequence having at least 90%, preferably 95%, 96%, 97%, 98%, 99%, or 100% identity with the nucleotide sequence and corresponding modifications set forth as SEQ ID NO: 15, and an antisense strand consisting of a nucleotide sequence having at least 90%, preferably 95%, 96%, 97%, 98%, 99%, or 100% identity with the nucleotide sequence and corresponding modifications set forth as SEQ ID NO: 16; (9) a sense strand consisting of a nucleotide sequence having at least 90%, preferably 95%, 96%, 97%, 98%, 99%, or 100% identity with the nucleotide sequence and corresponding modifications set forth as SEQ ID NO: 17, and an antisense strand consisting of a nucleotide sequence having at least 90%, preferably 95%, 96%, 97%, 98%, 99%, or 100% identity with the nucleotide sequence and corresponding modifications set forth as SEQ ID NO: 18; (10) a sense strand consisting of a nucleotide sequence having at least 90%, preferably 95%, 96%, 97%, 98%, 99%, or 100% identity with the nucleotide sequence and corresponding modifications set forth as SEQ ID NO: 19, and an antisense strand consisting of a nucleotide sequence having at least 90%, preferably 95%, 96%, 97%, 98%, 99%, or 100% identity with the nucleotide sequence and corresponding modifications set forth as SEQ ID NO: 20; (11) a sense strand consisting of a nucleotide sequence having at least 90%, preferably 95%, 96%, 97%, 98%, 99%, or 100% identity with the nucleotide sequence and corresponding modifications set forth as SEQ ID NO: 21, and an antisense strand consisting of a nucleotide sequence having at least 90%, preferably 95%, 96%, 97%, 98%, 99%, or 100% identity with the nucleotide sequence and corresponding modifications set forth as SEQ ID NO: 22; (12) a sense strand consisting of a nucleotide sequence having at least 90%, preferably 95%, 96%, 97%, 98%, 99%, or 100% identity with the nucleotide sequence and corresponding modifications set forth as SEQ ID NO: 23, and an antisense strand consisting of a nucleotide sequence having at least 90%, preferably 95%, 96%, 97%, 98%, 99%, or 100% identity with the nucleotide sequence and corresponding modifications set forth as SEQ ID NO: 24; (13) a sense strand consisting of a nucleotide sequence having at least 90%, preferably 95%, 96%, 97%, 98%, 99%, or 100% identity with the nucleotide sequence and corresponding modifications set forth as SEQ ID NO: 25, and an antisense strand consisting of a nucleotide sequence having at least 90%, preferably 95%, 96%, 97%, 98%, 99%, or 100% identity with the nucleotide sequence and corresponding modifications set forth as SEQ ID NO: 2; (14) a sense strand consisting of a nucleotide sequence having at least 90%, preferably 95%, 96%, 97%, 98%, 99%, or 100% identity with the nucleotide sequence and corresponding modifications set forth as SEQ ID NO: 26, and an antisense strand consisting of a nucleotide sequence having at least 90%, preferably 95%, 96%, 97%, 98%, 99%, or 100% identity with the nucleotide sequence and corresponding modifications set forth as SEQ ID NO: 4; (15) a sense strand consisting of a nucleotide sequence having at least 90%, preferably 95%, 96%, 97%, 98%, 99%, or 100% identity with the nucleotide sequence and corresponding modifications set forth as SEQ ID NO: 27, and an antisense strand consisting of a nucleotide sequence having at least 90%, preferably 95%, 96%, 97%, 98%, 99%, or 100% identity with the nucleotide sequence and corresponding modifications set forth as SEQ ID NO: 6; (16) a sense strand consisting of a nucleotide sequence having at least 90%, preferably 95%, 96%, 97%, 98%, 99%, or 100% identity with the nucleotide sequence and corresponding modifications set forth as SEQ ID NO: 28, and an antisense strand consisting of a nucleotide sequence having at least 90%, preferably 95%, 96%, 97%, 98%, 99%, or 100% identity with the nucleotide sequence and corresponding modifications set forth as SEQ ID NO: 8; (17) a sense strand consisting of a nucleotide sequence having at least 90%, preferably 95%, 96%, 97%, 98%, 99%, or 100% identity with the nucleotide sequence and corresponding modifications set forth as SEQ ID NO: 29, and an antisense strand consisting of a nucleotide sequence having at least 90%, preferably 95%, 96%, 97%, 98%, 99%, or 100% identity with the nucleotide sequence and corresponding modifications set forth as SEQ ID NO: 10; (18) a sense strand consisting of a nucleotide sequence having at least 90%, preferably 95%, 96%, 97%, 98%, 99%, or 100% identity with the nucleotide sequence and corresponding modifications set forth as SEQ ID NO: 30, and an antisense strand consisting of a nucleotide sequence having at least 90%, preferably 95%, 96%, 97%, 98%, 99%, or 100% identity with the nucleotide sequence and corresponding modifications set forth as SEQ ID NO: 12; (19) a sense strand consisting of a nucleotide sequence having at least 90%, preferably 95%, 96%, 97%, 98%, 99%, or 100% identity with the nucleotide sequence and corresponding modifications set forth as SEQ ID NO: 31, and an antisense strand consisting of a nucleotide sequence having at least 90%, preferably 95%, 96%, 97%, 98%, 99%, or 100% identity with the nucleotide sequence and corresponding modifications set forth as SEQ ID NO: 14; (20) a sense strand consisting of a nucleotide sequence having at least 90%, preferably 95%, 96%, 97%, 98%, 99%, or 100% identity with the nucleotide sequence and corresponding modifications set forth as SEQ ID NO: 32, and an antisense strand consisting of a nucleotide sequence having at least 90%, preferably 95%, 96%, 97%, 98%, 99%, or 100% identity with the nucleotide sequence and corresponding modifications set forth as SEQ ID NO: 16; (21) a sense strand consisting of a nucleotide sequence having at least 90%, preferably 95%, 96%, 97%, 98%, 99%, or 100% identity with the nucleotide sequence and corresponding modifications set forth as SEQ ID NO: 33, and an antisense strand consisting of a nucleotide sequence having at least 90%, preferably 95%, 96%, 97%, 98%, 99%, or 100% identity with the nucleotide sequence and corresponding modifications set forth as SEQ ID NO: 18; (22) a sense strand consisting of a nucleotide sequence having at least 90%, preferably 95%, 96%, 97%, 98%, 99%, or 100% identity with the nucleotide sequence and corresponding modifications set forth as SEQ ID NO: 34, and an antisense strand consisting of a nucleotide sequence having at least 90%, preferably 95%, 96%, 97%, 98%, 99%, or 100% identity with the nucleotide sequence and corresponding modifications set forth as SEQ ID NO: 20; (23) a sense strand consisting of a nucleotide sequence having at least 90%, preferably 95%, 96%, 97%, 98%, 99%, or 100% identity with the nucleotide sequence and corresponding modifications set forth as SEQ ID NO: 35, and an antisense strand consisting of a nucleotide sequence having at least 90%, preferably 95%, 96%, 97%, 98%, 99%, or 100% identity with the nucleotide sequence and corresponding modifications set forth as SEQ ID NO: 22; and (24) a sense strand consisting of a nucleotide sequence having at least 90%, preferably 95%, 96%, 97%, 98%, 99%, or 100% identity with the nucleotide sequence and corresponding modifications set forth as SEQ ID NO: 36, and an antisense strand consisting of a nucleotide sequence having at least 90%, preferably 95%, 96%, 97%, 98%, 99%, or 100% identity with the nucleotide sequence and corresponding modifications set forth as SEQ ID NO: 24.

In some embodiments, the nucleic acid sequence of the double-stranded RNAi molecule comprises one or more of the following items: (1) a sense strand with a nucleotide sequence consisting of SEQ ID NO: 1 and 0-5 additional nucleotides at the 5' and/or 3' ends, and an antisense strand with the nucleotide sequence consisting of SEQ ID NO: 2 and 0-5 additional nucleotides at the 5' and/or 3' ends; (2) a sense strand with a nucleotide sequence consisting of SEQ ID NO: 3 and 0-5 additional nucleotides at the 5' and/or 3' ends, and an antisense strand with the nucleotide sequence consisting of SEQ ID NO: 4 and 0-5 additional nucleotides at the 5' and/or 3' ends; (3) a sense strand with a nucleotide sequence consisting of SEQ ID NO: 5 and 0-5 additional nucleotides at the 5' and/or 3' ends, and an antisense strand with the nucleotide sequence consisting of SEQ ID NO: 6 and 0-5 additional nucleotides at the 5' and/or 3' ends; (4) a sense strand with a nucleotide sequence consisting of SEQ ID NO: 7 and 0-5 additional nucleotides at the 5' and/or 3' ends, and an antisense strand with the nucleotide sequence consisting of SEQ ID NO: 8 and 0-5 additional nucleotides at the 5' and/or 3' ends; (5) a sense strand with a nucleotide sequence consisting of SEQ ID NO: 9 and 0-5 additional nucleotides at the 5' and/or 3' ends, and an antisense strand with the nucleotide sequence consisting of SEQ ID NO: 10 and 0-5 additional nucleotides at the 5' and/or 3' ends; (6) a sense strand with a nucleotide sequence consisting of SEQ ID NO: 11 and 0-5 additional nucleotides at the 5' and/or 3' ends, and an antisense strand with the nucleotide sequence consisting of SEQ ID NO: 12 and 0-5 additional nucleotides at the 5' and/or 3' ends; (7) a sense strand with a nucleotide sequence consisting of SEQ ID NO: 13 and 0-5 additional nucleotides at the 5' and/or 3' ends, and an antisense strand with the nucleotide sequence consisting of SEQ ID NO: 14 and 0-5 additional nucleotides at the 5' and/or 3' ends; (8) a sense strand with a nucleotide sequence consisting of SEQ ID NO: 15 and 0-5 additional nucleotides at the 5' and/or 3' ends, and an antisense strand with the nucleotide sequence consisting of SEQ ID NO: 16 and 0-5 additional nucleotides at the 5' and/or 3' ends; (9) a sense strand with a nucleotide sequence consisting of SEQ ID NO: 17 and 0-5 additional nucleotides at the 5' and/or 3' ends, and an antisense strand with the nucleotide sequence consisting of SEQ ID NO: 18 and 0-5 additional nucleotides at the 5' and/or 3' ends; (10) a sense strand with a nucleotide sequence consisting of SEQ ID NO: 19 and 0-5 additional nucleotides at the 5' and/or 3' ends, and an antisense strand with the nucleotide sequence consisting of SEQ ID NO: 20 and 0-5 additional nucleotides at the 5' and/or 3' ends; (11) a sense strand with a nucleotide sequence consisting of SEQ ID NO: 21 and 0-5 additional nucleotides at the 5' and/or 3' ends, and an antisense strand with the nucleotide sequence consisting of SEQ ID NO: 22 and 0-5 additional nucleotides at the 5' and/or 3' ends; (12) a sense strand with a nucleotide sequence consisting of SEQ ID NO: 23 and 0-5 additional nucleotides at the 5' and/or 3' ends, and an antisense strand with the nucleotide sequence consisting of SEQ ID NO: 24 and 0-5 additional nucleotides at the 5' and/or 3' ends; (13) a sense strand with a nucleotide sequence consisting of SEQ ID NO: 25 and 0-5 additional nucleotides at the 5' and/or 3' ends, and an antisense strand with the nucleotide sequence consisting of SEQ ID NO: 2 and 0-5 additional nucleotides at the 5' and/or 3' ends; (14) a sense strand with a nucleotide sequence consisting of SEQ ID NO: 26 and 0-5 additional nucleotides at the 5' and/or 3' ends, and an antisense strand with the nucleotide sequence consisting of SEQ ID NO: 4 and 0-5 additional nucleotides at the 5' and/or 3' ends; (15) a sense strand with a nucleotide sequence consisting of SEQ ID NO: 27 and 0-5 additional nucleotides at the 5' and/or 3' ends, and an antisense strand with the nucleotide sequence consisting of SEQ ID NO: 6 and 0-5 additional nucleotides at the 5' and/or 3' ends; (16) a sense strand with a nucleotide sequence consisting of SEQ ID NO: 28 and 0-5 additional nucleotides at the 5' and/or 3' ends, and an antisense strand with the nucleotide sequence consisting of SEQ ID NO: 8 and 0-5 additional nucleotides at the 5' and/or 3' ends; (17) a sense strand with a nucleotide sequence consisting of SEQ ID NO: 29 and 0-5 additional nucleotides at the 5' and/or 3' ends, and an antisense strand with the nucleotide sequence consisting of SEQ ID NO: 10 and 0-5 additional nucleotides at the 5' and/or 3' ends; (18) a sense strand with a nucleotide sequence consisting of SEQ ID NO: 30 and 0-5 additional nucleotides at the 5' and/or 3' ends, and an antisense strand with the nucleotide sequence consisting of SEQ ID NO: 12 and 0-5 additional nucleotides at the 5' and/or 3' ends; (19) a sense strand with a nucleotide sequence consisting of SEQ ID NO: 31 and 0-5 additional nucleotides at the 5' and/or 3' ends, and an antisense strand with the nucleotide sequence consisting of SEQ ID NO: 14 and 0-5 additional nucleotides at the 5' and/or 3' ends; (20) a sense strand with a nucleotide sequence consisting of SEQ ID NO: 32 and 0-5 additional nucleotides at the 5' and/or 3' ends, and an antisense strand with the nucleotide sequence consisting of SEQ ID NO: 16 and 0-5 additional nucleotides at the 5' and/or 3' ends; (21) a sense strand with a nucleotide sequence consisting of SEQ ID NO: 33 and 0-5 additional nucleotides at the 5' and/or 3' ends, and an antisense strand with the nucleotide sequence consisting of SEQ ID NO: 18 and 0-5 additional nucleotides at the 5' and/or 3' ends; (22) a sense strand with a nucleotide sequence consisting of SEQ ID NO: 34 and 0-5 additional nucleotides at the 5' and/or 3' ends, and an antisense strand with the nucleotide sequence consisting of SEQ ID NO: 20 and 0-5 additional nucleotides at the 5' and/or 3' ends; (23) a sense strand with a nucleotide sequence consisting of SEQ ID NO: 35 and 0-5 additional nucleotides at the 5' and/or 3' ends, and an antisense strand with the nucleotide sequence consisting of SEQ ID NO: 22 and 0-5 additional nucleotides at the 5' and/or 3' ends; and (24) a sense strand with a nucleotide sequence consisting of SEQ ID NO: 36 and 0-5 additional nucleotides at the 5' and/or 3' ends, and an antisense strand with the nucleotide sequence consisting of SEQ ID NO: 24 and 0-5 additional nucleotides at the 5' and/or 3' ends.

In some embodiments, the double-stranded RNAi molecule is selected from one or more of the following groups: (1) a sense strand with the nucleotide sequence set forth as SEQ ID NO: 25, and an antisense strand with the nucleotide sequence set forth as SEQ ID NO: 2; (2) a sense strand with the nucleotide sequence set forth as SEQ ID NO: 26, and an antisense strand with the nucleotide sequence set forth as SEQ ID NO: 4; (3) a sense strand with the nucleotide sequence set forth as SEQ ID NO: 27, and an antisense strand with the nucleotide sequence set forth as SEQ ID NO: 6; (4) a sense strand with the nucleotide sequence set forth as SEQ ID NO: 28, and an antisense strand with the nucleotide sequence set forth as SEQ ID NO: 8; (5) a sense strand with the nucleotide sequence set forth as SEQ ID NO: 29, and an antisense strand with the nucleotide sequence set forth as SEQ ID NO: 10; (6) a sense strand with the nucleotide sequence set forth as SEQ ID NO: 30, and an antisense strand with the nucleotide sequence set forth as SEQ ID NO: 12; (7) a sense strand with the nucleotide sequence set forth as SEQ ID NO: 31, and an antisense strand with the nucleotide sequence set forth as SEQ ID NO: 14; (8) a sense strand with the nucleotide sequence set forth as SEQ ID NO: 32, and an antisense strand with the nucleotide sequence set forth as SEQ ID NO: 16; (9) a sense strand with the nucleotide sequence set forth as SEQ ID NO: 33, and an antisense strand with the nucleotide sequence set forth as SEQ ID NO: 18; (10) a sense strand with the nucleotide sequence set forth as SEQ ID NO: 34, and an antisense strand with the nucleotide sequence set forth as SEQ ID NO: 20; (11) a sense strand with the nucleotide sequence set forth as SEQ ID NO: 35, and an antisense strand with the nucleotide sequence set forth as SEQ ID NO: 22; and (12) a sense strand with the nucleotide sequence set forth as SEQ ID NO: 36, and an antisense strand with the nucleotide sequence set forth as SEQ ID NO: 24; wherein the 3'-OH at the 3' end of the sense strand SEQ ID NO: 25-36 is linked with an L96 ligand as shown in the following Formula I:

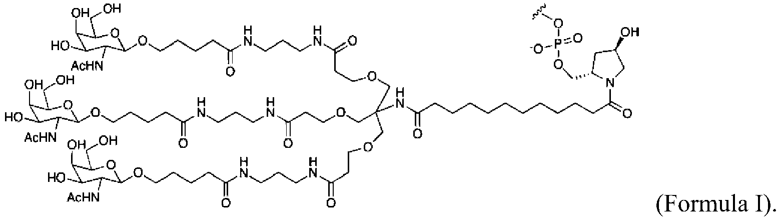

As used herein, the term "ligand" refers to the lipophilic moiety taken up by the host cell. Ligand modification can improve the cellular uptake, intracellular targeting, half-life, or medicament metabolism or pharmacokinetic properties of dsRNA molecules. In some embodiments, compared to unmodified dsRNAs, ligand-modified dsRNAs exhibit enhanced affinity or cellular uptake of selected targets (such as specific tissue types, cell types, organelles, etc.), such as hepatocytes. Ligand modification does not interfere with the activity of the dsRNA molecules.

In some embodiments, the ligand modification is to modify the 3' end, 5' end, and/or the middle of the sequence of the dsRNA molecule with one or more ligands.

In some preferred embodiments, the ligand is selected from the group consisting of: cholesterol, biotin, vitamin, galactose derivative or analog, lactose derivative or analog, N-acetylgalactosamine derivative or analog, and N-acetylglucosamine derivative or analog. The ligand is targeted at cell surface receptors, including galactose, galactosamine, lactose, or N-acetylgalactosamine/glucosamine moieties. Preferably, the ligand is targeted at the liver, especially the parenchymal cells of the liver.

In some preferred embodiments, the ligand is targeted at the ASGPR receptor.

In some preferred embodiments, the ligand may also be human serum albumin (HSA), hyaluronic acid, polypeptides, etc.

In some preferred embodiments, the ligand-modified dsRNA is characterized in that: the dsRNA molecule comprises a sense strand and an antisense strand, wherein
(1) the sense strand sequence is set forth as SEQ ID NO: 25, and the antisense strand sequence is set forth as SEQ ID NO: 2;
(2) the sense strand sequence is set forth as SEQ ID NO: 26, and the antisense strand sequence is set forth as SEQ ID NO: 4;
(3) the sense strand sequence is set forth as SEQ ID NO: 27, and the antisense strand sequence is set forth as SEQ ID NO: 6;
(4) the sense strand sequence is set forth as SEQ ID NO: 28, and the antisense strand sequence is set forth as SEQ ID NO: 8;
(5) the sense strand sequence is set forth as SEQ ID NO: 29, and the antisense strand sequence is set forth as SEQ ID NO: 10;
(6) the sense strand sequence is set forth as SEQ ID NO: 30, and the antisense strand sequence is set forth as SEQ ID NO: 12;
(7) the sense strand sequence is set forth as SEQ ID NO: 31, and the antisense strand sequence is set forth as SEQ ID NO: 14;
(8) the sense strand sequence is set forth as SEQ ID NO: 32, and the antisense strand sequence is set forth as SEQ ID NO: 16;
(9) the sense strand sequence is set forth as SEQ ID NO: 33, and the antisense strand sequence is set forth as SEQ ID NO: 18;
(10) the sense strand sequence is set forth as SEQ ID NO: 34, and the antisense strand sequence is set forth as SEQ ID NO: 20;
(11) the sense strand sequence is set forth as SEQ ID NO: 35, and the antisense strand sequence is set forth as SEQ ID NO: 22; or
(12) the sense strand sequence is set forth as SEQ ID NO: 36, and the antisense strand sequence is set forth as SEQ ID NO: 24;
wherein Am, Um, Cm, and Gm represent ribonucleotides A, U, C, and G that have been modified with 2'-O-methyl, respectively; Af, Uf, Cf, and Gf represent ribonucleotides A, U, C, and G that have been modified with 2'-fluoro, respectively; and (s) indicates that the two adjacent nucleotides are linked by a phosphorothioate backbone. The ligand is L96, and its structure is shown in the following Formula I:

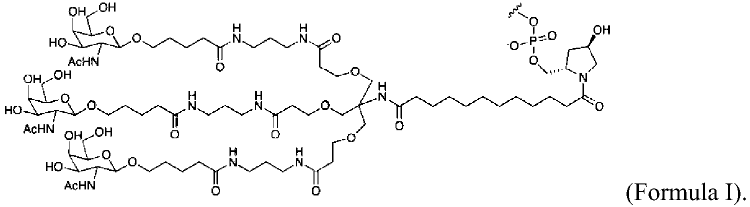

In some embodiments, the dsRNA has the structure shown in the following Formula II:

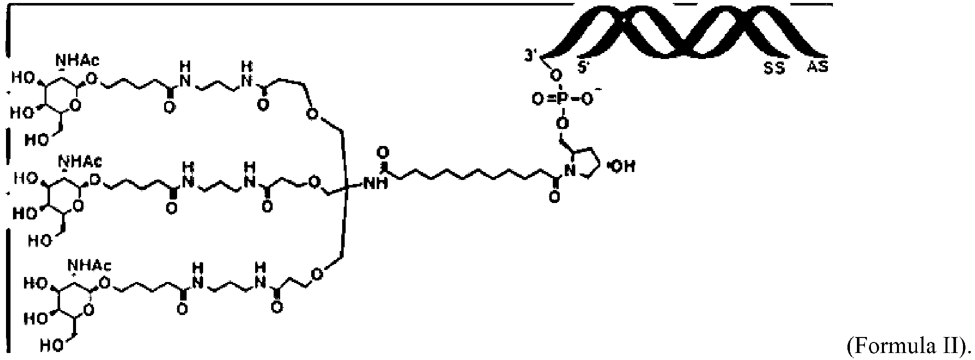

In some preferred embodiments, each strand of the dsRNA molecule may contain 0-100% modified nucleotides, such as 5%, 10%, 15%, 20%, 25%, 30%, 35%, 40%, 45%, 50%, 55%, 60%, 65%, 70%, 75%, 80%, 85%, 90%, 95%, 99%, or 100% modified nucleotides. The modifications can be applied to the overhang region or the double-chain region. The modifications can be used to improve the in vitro or in vivo characteristics of dsRNA molecules, such as stability, biodistribution, and inhibitory activity. The above modifications can be used in combination.

In some preferred embodiments, each strand of the dsRNA molecule has an overhang or blunt end. The 5' and/or 3' of any single chain or double chain have 1-8 overhangs, such as 1, 2, 3, 4, 5, 6, 7, or 8 overhangs, and the overhangs are arbitrarily selected from U, A, G, C, T, or dT.

In some preferred embodiments, the dsRNA molecule is able to suppress the expression of the MASP2 gene in human and cynomolgus monkey.

In another aspect, this application also relates to a biomaterial related to dsRNA;

In some embodiments, the dsRNA-associated biomaterial may be any one selected from the group consisting of:
(A) a DNA molecule capable of producing the dsRNA;
(B) a vector capable of expressing the dsRNA;
(C) a reagent or kit comprising the dsRNA or the DNA molecule or the vector;
(D) a pharmaceutical composition comprising the dsRNA molecule and other pharmaceutically acceptable components.

In some embodiments, the pharmaceutical composition comprises a pharmacologically effective amount of the dsRNA molecule of this application and other pharmaceutically acceptable components. The term "effective amount" refers to the amount of dsRNA molecules that can effectively produce the expected pharmacological therapeutic effect.

In some embodiments, "other components" comprise water, saline, glucose, buffer (such as PBS), excipient, diluent, disintegrant, binder, lubricant, sweetener, flavoring agent, preservative, or combinations thereof.

In another aspect, the dsRNA or related biomaterials involved in this application can be used to prevent and/or treat a disease mediated by the MASP2 gene, or to alleviate the symptoms of a disease mediated by the MASP2 gene.

A disease mediated by the MASP2 gene include, but are not limited to: paroxysmal nocturnal hemoglobinuria (PNH), atypical hemolytic uremic syndrome (aHUS), asthma, rheumatoid arthritis (RA), antiphospholipid antibody syndrome, lupus nephritis, ischemia-reperfusion injury, typical or infectious hemolytic uremic syndrome (tHUS), dense deposit disease (DDD), neuromyelitis optica (NMO), multifocal motor neuropathy (MMN), multiple sclerosis (MS), macular degeneration (e.g., age-related macular degeneration (AMD)), hemolysis, elevated liver enzymes and low platelet count (HELLP) syndrome, thrombotic thrombocytopenic purpura (TTP); spontaneous abortion, oligoimmune vasculitis, epidermolysis bullosa, recurrent abortion, preeclampsia, traumatic brain injury, myasthenia gravis, cold agglutinin disease, dermatomyositis, bullous pemphigoid, and Shiga toxin *Escherichia coli* (*E. coli*)-associated hemolytic uremic syndrome, C3 nephropathy, anti-neutrophil cytoplasmic antibody-associated vasculitis, humoral and vascular transplant rejection, graft dysfunction, myocardial infarction, xenograft, sepsis, coronary artery disease, dermatomyositis, Graves' disease, atherosclerosis, Alzheimer's disease, systemic inflammatory response sepsis, septic shock, spinal cord injury, glomerulonephritis, Hashimoto's thyroiditis, type I diabetes, psoriasis, pemphigus, autoimmune hemolytic anemia (AIHA), ITP. Pulmonary hemorrhage nephritis syndrome, Degos disease, antiphospholipid syndrome (APS), catastrophic APS (CAPS), cardiovascular disease, myocarditis, cerebrovascular disease, peripheral vascular disease, renal vascular disease, mesenteric/enterovascular disease, vasculitis, Henlein-Schönlein purpura nephritis, vasculitis associated with systemic lupus erythematosus, vasculitis associated with rheumatoid arthritis, immune complex vasculitis, Takayau's disease, dilated cardiomyopathy, diabetic angiopathy, Kawasaki disease (arteritis), diseases associated with viral infection (such as COVID-19), chronic obstructive pulmonary disease (COPD), acute respiratory distress syndrome (ARDS), tumors associated with MASP2 (e.g., liver cancer, lung cancer), venous gas embolism (VGE), and stent placement, rotational atherosclerotic plaque removal, membranous nephropathy, Guillain-Barre syndrome, and restenosis after percutaneous transluminal coronary angioplasty (PTCA).

In some embodiments, this application also provides use selected from any of the following: use of the dsRNA or the biomaterial in inhibiting MASP2 gene expression or in the preparation of a product for inhibiting MASP2 gene expression. The inhibition of MASP2 gene expression refers to inhibiting or reducing the expression level of the MASP2 gene in in vivo or in vitro cells of human and cynomolgus monkey. The inhibition of MASP2 gene expression means that the expression level of MASP2 gene is inhibited or reduced by at least 95%, 90%, 85%, 80%, 75%, 70%, 60%, 50%, 40%, 30%, 20%, 10%, or 5%. Detection of target gene, target RNA, or target protein levels can be used to predict or assess activity, efficacy, or treatment outcomes.

In some embodiments, the cells are mammalian cells expressing MASP2, such as primate cells or human cells. More preferably, the target cells show high levels of MASP2 gene expression. More preferably: the cells are derived from the brain, salivary gland, heart, spleen, lung, liver, kidney, intestine, and tumor. Still more preferably: the cells are liver cancer cells.

In some embodiments, the cells are selected from the group consisting of: HepG2, HEP3B, Huh7, MHCC97H, Hela, cynomolgus monkey primary cells, and human primary cells.

In some embodiments, the final cellular concentration of the dsRNA molecule is 0.001-1000 nM, such as 0.001-10 nM, 10-500 nM, 25-300 nM or 50-100 nM.

In some embodiments, the dsRNA or associated biomaterial may be administered by any suitable means, such as parenteral administration, including intramuscular, intravenous, arterial, peritoneal, or subcutaneous injection. The administration manner includes, but is not limited to, single or multiple administrations.

In some preferred embodiments, the dosage range is 0.1-100 mg/kg, 0.5-50 mg/kg, 3-36 mg/kg, 2.5-20 mg/kg, and 5-15 mg/kg, for example: 3 mg/kg, 4 mg/kg, 5 mg/kg, 6 mg/kg, 7 mg/kg, 8 mg/kg, 9 mg/kg, 10 mg/kg, 11 mg/kg, 12 mg/kg, 13 mg/kg, 14 mg/kg, 15 mg/kg, 16 mg/kg, 17 mg/kg, 18 mg/kg, 19 mg/kg, 20 mg/kg, 21 mg/kg, 22 mg/kg, 23 mg/kg, 24 mg/kg, 25 mg/kg, 26 mg/kg, 27 mg/kg, 28mg/kg, 29mg/kg, 30mg/kg, 31mg/kg, 32mg/kg, 33mg/kg, 34mg/kg, 35mg/kg, or 36mg/kg.

In some embodiments, a single dose of the pharmaceutical composition can have a long duration, with the decrease in MASP2 expression lasting for at least 3, 5, 7, 10, 14 days or longer.

In some embodiments, provided is use of the dsRNA or the biomaterial in reducing serum MASP2 or in the preparation of a product for reducing serum MASP2. The reduction of serum MASP2 concentration refers to the reduction of MASP2 concentration in the serum of human and cynomolgus monkey. For example, the concentration or content of serum MASP2 is reduced by at least 5%, 10%, 15%, 20%, 25%, 30%, 35%, 40%, 50%, 60%, 70%, 80%, 90%, 95%, or 98%.

In some embodiments, provided is use of the dsRNA or the biomaterial in preventing and/or treating a disease mediated by the MASP2 gene or in the preparation of a product for preventing and/or treating a disease mediated by the MASP2 gene.

In some embodiments, provided is use of the dsRNA or the biomaterial in alleviating symptoms of a disease mediated by the MASP2 gene or in the preparation of a product for alleviating symptoms of a disease mediated by the MASP2 gene.

In some embodiments, preferably the disease mediated by the MASP2 gene is a cardiovascular disease, dyslipidemia, or neoplastic disease.

In some embodiments, the disease or symptoms mediated by the MASP2 gene may be caused by overexpression of the MASP2 gene or overproduction of the MASP2 protein, and may be regulated by downregulating MASP2 gene expression. The treatment refers to the alleviation, reduction, or cure of the disease or symptoms mediated by the MASP2 gene, such as a decrease in serum MASP2 level. For example, the concentration or content of of serum MASP2 is decreased by 5%, 10%, 15%, 20%, 25%, 30%, 35%, 40%, 50%, 60%, 70%, 80%, 90%, and 95%.

In some embodiments, also provided is use of the ligand-modified dsRNA molecules in the preparation of a liver-targeted medicament; wherein the liver-targeted medicament can be used for treating a liver disease mediated by the MASP2 gene.

In another aspect, this application also provides methods and/or combination therapies for treating subjects suffering from conditions that would benefit from suppression or reduction of MASP2 gene expression, for example, complement component MASP2-related diseases, such as paroxysmal nocturnal hemoglobinuria (PNH) and atypical hemolytic uremic syndrome (aHUS). These methods and/or combination therapies utilize lysed RNAi compositions mediated by RNA-induced silencing complex (RISC) affecting RNA transcripts of complement component MASP2 gene.

These combination therapies of this application comprise administering to a patient with a disease associated with a complement component MASP2 an RNAi agent of this application and an additional therapeutic agent, such as an anti-complement component MASP2 antibody or an antigen-binding fragment thereof, for example, narsoplimab. These combination therapies of the application reduce MASP2 level (e.g., approximately 30%, 35%, 40%, 45%, 50%, 55%, 60%, 65%, 70%, 75%, 80%, 85%, 90%, 95%, or approximately 99%), in a subject by targeting MASP2 mRNA at a RNAi agent of this application, and thus allow for a reduction in the therapeutic (or preventative) effective amount of eculizumab required to treat the subject, thereby reducing treatment costs and allowing for simpler and more convenient methods of administering eculizumab, such as subcutaneous administration.

In some embodiments, the additional therapeutic agent may be an anti-complement component MASP2 antibody or an antigen-binding fragment or derivative thereof.

In some embodiments, the anti-complement component MASP2 antibody is narsoplimab or an antigen-binding fragment or derivative thereof.

### The terms:

REL (Relative expression level): relative expression level of mRNA.

"G", "C", "A", "T" and "U" usually represent nucleotides with guanine, cytosine, adenine, thymine and uracil as bases, respectively.

N: refers to ribonucleotides, including: ribonucleotides are divided into adenine ribonucleotides, guanine ribonucleotides, cytosine ribonucleotides, and uracil ribonucleotides.
dN: refers to deoxyribonucleotides.
GalNAc: N-acetylgalactosamine
Nm=2'OMe modified ribonucleotides;
Nf=2'F modified ribonucleotides;
The (s) = PS backbone in the nucleotide sequence, and it is the 5'-thio-modified phosphate backbone;
DNA modification: the DNA modification of dsRNA described in this application refers to replacing the ribonucleotides in the dsRNA with deoxyribonucleotides, wherein the nucleotides are the same, only the types of ribose are different.

CAPA: capping reagent A (20% acetic anhydride in acetonitrile solution, v/v).

CAPB: capping reagent B (N-methylimidazole:pyridine:acetonitrile = 2:3:5).

ACN: acetonitrile.

TEAA: triethylamine acetic acid.

Trityl-off synthesis: synthesis of trityl-off.
ESI-MS: electrospray mass spectrometry;
IEX HPLC: ion-exchange high-performance liquid chromatography;
GAPDH: glyceraldehyde-3-phosphate dehydrogenase;
Mock group: negative control group, i.e., the control group of transfection reagent;

As used herein, the percentage of "identity," such as 85%, 90%, 91%, 92%, 93%, 94%, 95%, 96%, 97%, 98%, 98.5%, 99%, and 99.5% identity, refers to the degree of similarity between amino acid sequences or nucleotide sequences as determined by sequence alignment. For example, by introducing gaps or other methods, two sequences can be made to have the same residues at as many positions as possible, and the proportion of positions with the same bases or amino acid residues to the total number of positions can be determined. The percentage of "identity" can be determined by using software programs known in the art. It is preferable to use the default parameters for comparison. A preferred alignment program is BLAST. The preferred programs are BLASTN and BLASTP. Details of these programs can be found on the corresponding pages of the NCBI website. It is particularly important to note that, when describing a nucleotide sequence that has at least a certain percentage identity with a certain nucleotide sequence and the corresponding modifications (e.g., "a nucleotide sequence that has at least 90% identity with the nucleotide sequence UmsAmsGmCmUmGmUfAmGfAfAfAmUmGmUmAmUmCmCmsUmsGm (SEQ ID NO: 5) and the corresponding modifications"), the sequence alignment must take into account the modifications of each nucleotide monomer of the nucleotide sequence. That is, the two nucleotides are considered to be the same nucleotide only when the entire monomer (comprising artificial modifications) of the two nucleotides is completely identical.

As used herein, the "complementarity" of nucleic acids refers to the ability of one nucleic acid to form hydrogen bonds with another nucleic acid through traditional Watson-Crick base pairing. Percentage complementarity refers to the percentage of nucleotides in the shorter nucleic acid molecule that can form hydrogen bonds (i.e. Watson Crick base pairing) with the other nucleic acid molecule among all nucleotides in the shorter nucleic acid molecule (e.g., approximately 5, 6, 7, 8, 9, and 10 out of 10 are approximately 50%, 60%, 70%, 80%, 90%, and 100% complementary, respectively). "Complete complementarity" means that all consecutive residues in a nucleic acid sequence form hydrogen bonds with the same number of consecutive residues in a second nucleic acid sequence. As used herein, "substantially complementary" means a degree of complementarity of any one of at least 70%, 75%, 80%, 85%, 90%, 95%, 96%, 97%, 98%, 99%, or 100% complementary in a region of about 40, 50, 60, 70, 80, 100, 150, 200, 250 or more nucleotides, or refers to two nucleic acids that hybridize under stringent conditions. For a single base or a single nucleotide, according to the Watson-Crick base pairing rule, when A is paired with T or U, or C is paired with G or I, it is called complementary, paired, or matched, and vice versa; all other base pairings are called non-complementary.

As used herein, nucleic acid "hybridization" refers to a reaction in which one or more polynucleotides react to form a complex that is stabilized by hydrogen bonds between nucleotide residues. Hydrogen bonds can occur through Watson-Crick base pairing, Hoogstein binding, or any other sequence-specific mechanism. The complex may comprise two chains forming a double-stranded structure, three or more chains forming a multi-stranded complex, a single self-hybridized chain, or a combination thereof.

The term "nucleotide" herein refers not only to naturally occurring ribonucleotide or deoxyribonucleotide monomers, but also to their associated structural variants, including derivatives and analogs, which are functionally equivalent in relation to the specific context in which the nucleotide is used, unless the context explicitly indicates otherwise. For example, "nucleotide" refers to deoxyribonucleotide or ribonucleotide. Nucleotides can be standard nucleotides (i.e., adenosine, guanosine, cytidine, thymidine, and uridine), nucleotide isomers, or nucleotide analogs. Nucleotide analogs refer to nucleotides with modified purine or pyrimidine bases or modified ribose moieties. Nucleotide analogs can be naturally occurring nucleotides (such as inosine, pseudouridine, etc.) or non-naturally occurring nucleotides. Non-limiting examples of modifications to the sugar or base moiety of a nucleotide comprise the addition (or removal) of acetyl, amino, carboxyl, carboxymethyl, hydroxy, methyl, phosphoryl, and thiol groups, as well as the substitution of the carbon and nitrogen atoms of the base by other atoms (e.g., 7-denitropurine). Nucleotide analogs also comprise dideoxynucleotides, 2'-O-methylnucleotides, locked nucleic acids (LNA), peptide nucleic acids (PNA), and morpholino oligonucleotides. In some embodiments, the "nucleotide" in this application does not contain non-natural nucleotides with modified bases. In some embodiments, as used herein the term "nucleotide" does not comprise nucleotides with modified bases. In this application, "G", "C", "A", "T" and "U" generally represent nucleotides with guanine, cytosine, adenine, thymine and uracil as bases, respectively. However, in the context of RNA and in RNA sequences, unless otherwise specified, "T" refers to uridine or uracil. It should be understood that in the context of nucleotide sequences in this application, the terms "nucleotide," "nucleotide residue," and "base" are used interchangeably. The number of base pairs is measured in bp, and one bp is one base pair. The number of nucleotides is measured in nt, with one nt equal to one nucleotide.

As used herein, "3' end" particularly refers to the position of the first nucleotide or the first base pair at the 3' end of a single nucleotide sequence or a double-stranded polynucleotide. Therefore, "3' end" can be used interchangeably with "3' end nucleotide". The term "5' end" particularly refers to the position of the first nucleotide or the first base pair at the 5' end of a single nucleotide sequence or a double-stranded polynucleotide. Therefore, "5' end" can be used interchangeably with "5' end nucleotide".

As used herein, the term "nucleic acid molecule" may be used to refer to any molecule having a nucleotide sequence consisting of two or more nucleotides linked by a phosphate backbone, or a modified phosphate backbone (e.g., a phosphorothioate backbone).

The term "complement component MASP2" or "complement MASP2", which is used interchangeably with the term "MASP2", refers to the well-known gene and polypeptide, which is also known in the art as MAP-2, MAP19, MASP2, MASP1P1, sMAP; the sequence of the human MASP2 mRNA transcript can be found, for example, in the Genebank accession number GI: 1653961883 (NM_006610.4). The sequence of rhesus monkey MASP2 mRNA can be found, for example, in the gene bank accession number GI:1622835000(XM_001118827.4). The sequence of mouse MASP2 mRNA can be found, for example, in the gene bank accession number GI:90962989 (NM_001003893.2). The sequence of rat MASP2 mRNA can be found, for example, in the gene bank accession number GI:78042604 (NM_172043.1). Additional instances of the MASP2 mRNA sequence can be easily obtained by publicly available databases such as gene banks.

Typically, most nucleotides in each strand of a dsRNA molecule are ribonucleotides, but as described in detail herein, each strand or both strands may also comprise one or more non-ribonucleotides, such as deoxyribonucleotides and/or modified nucleotides. Additionally, as used in this specification, "RNAi reagent" may comprise chemically modified ribonucleotides; RNAi reagent may comprise numerous modifications on multiple nucleotides. Such modifications may comprise all types of modifications disclosed herein or known in the art. Any such modifications used in siRNA-type molecules are covered in the term "RNAi reagent" for the purposes of this specification and the claims.

As used herein, siRNA, dsRNA, or RNAi reagent have similar meanings and can be used interchangeably.

As used herein, unless otherwise indicated, the singular forms "a," "an," and "the" comprise the plural forms.

Unless otherwise defined herein, all technical and scientific terms used herein have the same meaning as commonly understood by one of ordinary skill in the art to which this application pertains. Unless otherwise stated, the practice of this application will employ conventional techniques of cell biology, cell culture, molecular biology, transgenic biology, microbiology, recombinant DNA, and immunology.

It should be understood that, this application comprises the various aspects, embodiments, and combinations of the aspects and/or embodiments described herein. The above description and the following Examples are intended to illustrate, and not limit, the scope of this application. Within the scope of the technical concept of this application, various simple modifications can be made to the technical solution of this application, including combining the various technical features in any other suitable manner. These simple modifications and combinations should also be regarded as the content disclosed in this application, and are all within the protection scope of this application.

### EXAMPLES

### Example 1: MASP2-dsRNA Activity Screening Assay

### 1.1 dsRNA Design

Based on the human MASP2 gene (Gene ID, 104710) mRNA sequence (NM_006610.4), 100 MASP2 dsRNAs (i.e., dsRNAs No. 1-100) were designed at different selected sites. Some naked or unmodified dsRNA sequences are shown in Table 1. The sequences were compared with all other non-target gene sequences by sequence similarity software and showed the lowest homology.

**Table 1: Some dsRNA sequences from high-throughput screening**

| SEQ ID NO: | Names of sequences | Sequences (5'->3') |
|---|---|---|
| 37 | Sense strand No. 9 | GCUCCGACUACUCCAACGAGA |
| 38 | Antisense strand No. 9 | UCUCGUUGGAGUAGUCGGAGCGG |
| 39 | Sense strand No. 10 | UAUCCGAUGGCGCCACCUAAU |
| 40 | Antisense strand No. 10 | AUUAGGUGGCGCCAUCGGAUAAG |
| 41 | Sense strand No. 11 | CACCGUAACAAGCGCACCUGA |
| 42 | Antisense strand No. 11 | UCAGGUGCGCUUGUUACGGUGCA |
| 43 | Sense strand No. 12 | CUACCGCCUGCGCCUCUACUU |
| 44 | Antisense strand No. 12 | AAGUAGAGGCGCAGGCGGUAGCC |
| 45 | Sense strand No. 13 | CCGUAACAAGCGCACCUGCUA |
| 46 | Antisense strand No. 13 | UAGCAGGUGCGCUUGUUACGGUG |
| 47 | Sense strand No. 14 | CCUCUGCGAGUACGACUUCGU |
| 48 | Antisense strand No. 14 | ACGAAGUCGUACUCGCAGAGGUG |
| 49 | Sense strand No. 15 | UGCGAGUACGACUUCGUCAAA |
| 50 | Antisense strand No. 15 | UUUGACGAAGUCGUACUCGCAGA |
| 51 | Sense strand No. 16 | GCCUGGACAUUACCUUCCGCU |
| 52 | Antisense strand No. 16 | AGCGGAAGGUAAUGUCCAGGCUG |
| 53 | Sense strand No. 17 | GAAGCCGUUCACGGGGUUCGA |
| 54 | Antisense strand No. 17 | UCGAACCCCGUGAACGGCUUCUC |
| 55 | Sense strand No. 18 | UACGUCCUGCACCGUAACAAA |
| 56 | Antisense strand No. 18 | UUUGUUACGGUGCAGGACGUAGC |
| 57 | Sense strand No. 20 | CUGCACCGUAACAAGCGCACC |
| 58 | Antisense strand No. 20 | GGUGCGCUUGUUACGGUGCAGGA |
| 59 | Sense strand No. 21 | UCUGCGAGUACGACUUCGUCA |
| 60 | Antisense strand No. 21 | UGACGAAGUCGUACUCGCAGAGG |
| 61 | Sense strand No. 22 | AGCCUGGACAUUACCUUCCGC |
| 62 | Antisense strand No. 22 | GCGGAAGGUAAUGUCCAGGCUGG |
| 63 | Sense strand No. 24 | GCUACGUCCUGCACCGUAACA |
| 64 | Antisense strand No. 24 | UGUUACGGUGCAGGACGUAGCCU |
| 65 | Sense strand No. 27 | UGGACAUUACCUUCCGCUCCG |
| 66 | Antisense strand No. 27 | CGGAGCGGAAGGUAAUGUCCAGG |
| 67 | Sense strand No. 28 | CUCCGACUACUCCAACGAGAA |
| 68 | Antisense strand No. 28 | UUCUCGUUGGAGUAGUCGGAGCG |
| 69 | Sense strand No. 29 | AGAAGCCGUUCACGGGGUUCG |
| 70 | Antisense strand No. 29 | CGAACCCCGUGAACGGCUUCUCG |
| 71 | Sense strand No. 32 | AGCUUCUCCAUCUUUUGCGAA |
| 72 | Antisense strand No. 32 | UUCGCAAAAGAUGGAGAAGCUGU |
| 73 | Sense strand No. 37 | CCUGGACAUUACCUUCCGCUC |
| 74 | Antisense strand No. 37 | GAGCGGAAGGUAAUGUCCAGGCU |
| 75 | Sense strand No. 38 | UCCGACUACUCCAACGAGAAG |
| 76 | Antisense strand No. 38 | CUUCUCGUUGGAGUAGUCGGAGC |
| 77 | Sense strand No. 40 | UGCACCGUAACAAGCGCACCU |
| 78 | Antisense strand No. 40 | AGGUGCGCUUGUUACGGUGCAGG |
| 79 | Sense strand No. 56 | CGUCCUGCACCGUAACAAGCG |
| 80 | Antisense strand No. 56 | CGCUUGUUACGGUGCAGGACGUA |
| 81 | Sense strand No. 60 | AUCCGAUGGCGCCACCUAAUG |
| 82 | Antisense strand No. 60 | CAUUAGGUGGCGCCAUCGGAUAA |
| 83 | Sense strand No. 63 | CUGCACCCCCCGGCUACCGCC |
| 84 | Antisense strand No. 63 | GGCGGUAGCCGGGGGGUGCAGUC |
| 85 | Sense strand No. 64 | UAACUAUAUUCCCUGGAUCGA |
| 86 | Antisense strand No. 64 | UCGAUCCAGGGAAUAUAGUUAAU |
| 87 | Sense strand No. 70 | AGGCUACGUCCUGCACCGUAA |
| 88 | Antisense strand No. 70 | UUACGGUGCAGGACGUAGCCUGC |
| 89 | Sense strand No. 71 | GGCUACGUCCUGCACCGUAAC |
| 90 | Antisense strand No. 71 | GUUACGGUGCAGGACGUAGCCUG |
| 91 | Sense strand No. 84 | AAGCCGUUCACGGGGUUCGAG |
| 92 | Antisense strand No. 84 | CUCGAACCCCGUGAACGGCUUCU |
| 93 | Sense strand No. 87 | UGUGUGGCUCGGUGGCCACCC |
| 94 | Antisense strand No. 87 | GGGUGGCCACCGAGCCACACAGA |
| 95 | Sense strand No. 91 | CUUAUCCGAUGGCGCCACCUA |
| 96 | Antisense strand No. 91 | UAGGUGGCGCCAUCGGAUAAGGG |
| 97 | Sense strand No. 92 | UCCGAUGGCGCCACCUAAUGG |
| 98 | Antisense strand No. 92 | CCAUUAGGUGGCGCCAUCGGAUA |
| 99 | Sense strand No. 93 | CCGAUGGCGCCACCUAAUGGC |
| 100 | Antisense strand No. 93 | GCCAUUAGGUGGCGCCAUCGGAU |
| 101 | Sense strand No. 99 | GAGUGGAGUACAUCACAGGUC |
| 102 | Antisense strand No. 99 | GACCUGUGAUGUACUCCACUCGG |

### 1.2 Synthesis and Purification of dsRNA (conjugates)

The dsRNA in this application comprises only ribonucleotides or oligonucleotides modified with 2'-methoxy or 2'-fluorine. It was synthesized according to the theoretical yield of 1 µmol. All oligonucleotides were prepared on an LK-192X synthesizer by using a 1 µmol universal Frit solid support (1000Å=100nm, Biocomma) or a controllable microporous glass (CPG) solid support (Asymchem) of GalNAc derivative L96 with protective groups. According to the needs of the sequences, all phosphoramidite monomers corresponding to the nucleoside (e.g., natural ribonucleotide monomers, 2'-OMe modified phosphoramidite nucleotide monomers, 2'-F modified phosphoramidite nucleotide monomers) were diluted with anhydrous acetonitrile as a solvent at a ratio of 1:40 (g/mL), and the coupling time was 3 min, for a total of two couplings. Deprotection was performed by using 3% trichloroacetic acid (TCA), activation was performed by using 0.3M benzylthiotetrazole acetonitrile solution, and capping and oxidation were performed by using CAPA/CAPB and 50mM I₂ solution, respectively. After the trityl-off synthesis, the solid support was transferred to a 2 mL centrifuge tube, and 1.2 mL of ammonia was added in the tube to heat in a 65 °C oven for 3 h to remove the protecting group, then cooling to room temperature, concentrating under vacuum for 30 min, filtering the solution through a 0.22 µm filter membrane into a sample vial. Single-chain purification was performed by using a semi-preparative reversed-phase purifier, with an elution gradient of 7%-30% (ACN: 100 mM TEAA) for 10 min at a flow rate of 5 mL/min. After purification, the resulting solution was concentrated under vacuum, then rotary evaporation to dryness at room temperature. Finally, the sample was dissolved in water, and each solution was desalted on a GE Hi-Trap desalting column to elute the final oligonucleotide product. All properties and purity were confirmed by using ESI-MS and IEX HPLC, respectively. The concentration was determined by ultraviolet light using an enzyme-linked immunosorbent assay (ELISA) reader. Equimolar amounts of sense and antisense strands were mixed and transferred to a new delivery tube. The mixture was heated at 95°C for 5 min, and then slowly annealed to room temperature. Finally, the final product was obtained by rotary evaporation to dryness at room temperature with a vacuum concentrator.

### 1.3 High-throughput Screening and Detection of MASP2-dsRNA In Vitro Activity

### 1.3.1 Transfection of HepG2 Cells with MASP2 dsRNA

HepG2 cells were cultured in DMEM medium (Gibco) containing 10% fetal bovine serum in a 5% CO₂ incubator at 37°C. When the cells were in the logarithmic growth phase and in good condition (70% confluence), they were plated and transfected. The cell density was adjusted to 2 × 10⁵ cells per well in a 24-well plate to incubate overnight in a 5% CO₂ incubator at 37°C. Preparation of transfection complex: 250 µL of Opti-MEM (Gibco) and 5 µL of 1 nM or 0.1 nM dsRNA were mixed, 250 µL of Opti-MEM and 1.5 µL of Lipofectamine^{™} RNAiMax transfection reagent (Thermo Fisher Scientific) were mixed, after standing for 5 min, then mixing the two mixtures together and standing for 20 min. The MEM medium in the 24-well plate was sucked to discard, then adding the above transfection complex to each well to incubate in a 5% CO₂ incubator at 37°C for 6 hours. The supernatant was sucked out from the 24-well plate, then adding 1 mL of complete culture medium (DMEM + 10% FBS) to each well, and culturing continuously in a 5% CO₂ incubator at 37°C for 24 h.

For each time of cell plating, a control group for transfection reagents was also set up in addition to the experimental group. Both the experimental and control groups were replicated three times.

### 1.3.2 Real-time Quantitative PCR Analysis of Target mNRA Levels

24 hours after transfection, the above transfected HepG2 cells were lysed, and total RNA was extracted from the cells by Vazyme FastPure Cell/Tissue Total RNA Isolation Kit V2 (refer to the Vazyme RC112-01 instruction manual). The RNA was reverse transcribed into cDNA by using Takara PrimeScript RT Master Mix RR036Q. Table 2 shows the primer sequence information for q-PCR. The human GAPDH gene (NCBI accession number: NM_002046.7) was used as the internal reference gene, and PCR reactions were performed by using a Bio-Rad CFX96 real-time q-PCR instrument. In the experiment, the Mock group (i.e., the negative control group) was used as the control for normalization treatment, and the MASP2 mRNA expression level in the Mock group was recorded as 1.

**Table 2: qPCR primer sequence information**

| Names of primers | Upstream primer sequence (5' to 3') | Downstream primer sequence (5' to 3') |
|---|---|---|
| Human-GAPDH | GATTTGGTCGTATTGGGCGC (SEQ ID NO: 103) | TTCCCGTTCTCAGCCTTGAC (SEQ ID NO: 104) |
| Human-MASP2 | CCCAGTCTGTGAGCCTGTTT (SEQ ID NO: 105) | GCAGCTGTTAGGACCCAGTT (SEQ ID NO: 106) |

### 1.3.3 Data Analysis

After the PCR reaction, the internal reference gene (human GAPDH gene) was used as the standard for relative quantitative analysis by CFX96 software, and statistical analysis was performed by GarphPad software. Table 3 shows the high-throughput screening and validation results of dsRNA molecules in HepG2 cells.

**Table 3: Results of high-throughput screening in HepG2 cells with 1 nM single dose**

| No. | Relative activity | Mean error ±SEM |
|---|---|---|
| 1 | 0.470 | 0.057 |
| 2 | 0.348 | 0.052 |
| 3 | 0.454 | 0.101 |
| 4 | 0.417 | 0.081 |
| 5 | 0.338 | 0.009 |
| 6 | 0.330 | 0.035 |
| 7 | 0.504 | 0.031 |
| 8 | 0.326 | 0.014 |
| 9 | 0.166 | 0.010 |
| 10 | 0.140 | 0.016 |
| 11 | 0.169 | 0.032 |
| 12 | 0.215 | 0.048 |
| 13 | 0.262 | 0.038 |
| 14 | 0.186 | 0.006 |
| 15 | 0.205 | 0.066 |
| 16 | 0.185 | 0.044 |
| 17 | 0.156 | 0.013 |
| 18 | 0.246 | 0.035 |
| 19 | 0.482 | 0.181 |
| 20 | 0.391 | 0.072 |
| 21 | 0.586 | 0.116 |
| 22 | 0.500 | 0.122 |
| 23 | 0.634 | 0.110 |
| 24 | 0.433 | 0.025 |
| 25 | 0.739 | 0.040 |
| 26 | 0.344 | 0.046 |
| 27 | 0.348 | 0.021 |
| 28 | 0.316 | 0.019 |
| 29 | 0.431 | 0.004 |
| 30 | 0.363 | 0.020 |
| 31 | 0.344 | 0.012 |
| 32 | 0.183 | 0.032 |
| 33 | 0.390 | 0.034 |
| 34 | 0.350 | 0.011 |
| 35 | 0.421 | 0.035 |
| 36 | 0.368 | 0.048 |
| 37 | 0.521 | 0.077 |
| 38 | 0.514 | 0.079 |
| 39 | 0.390 | 0.020 |
| 40 | 0.213 | 0.019 |
| 41 | 0.511 | 0.036 |
| 42 | 0.354 | 0.073 |
| 43 | 0.543 | 0.075 |
| 44 | 0.427 | 0.082 |
| 45 | 0.468 | 0.112 |
| 46 | 0.581 | 0.080 |
| 47 | 0.550 | 0.010 |
| 48 | 0.474 | 0.028 |
| 49 | 0.336 | 0.061 |
| 50 | 0.514 | 0.100 |
| 51 | 0.468 | 0.042 |
| 52 | 0.398 | 0.012 |
| 53 | 0.552 | 0.004 |
| 54 | 0.496 | 0.060 |
| 55 | 0.271 | 0.030 |
| 56 | 0.304 | 0.006 |
| 57 | 0.442 | 0.010 |
| 58 | 0.335 | 0.024 |
| 59 | 0.564 | 0.190 |
| 60 | 0.422 | 0.032 |
| 61 | 0.534 | 0.027 |
| 62 | 0.431 | 0.021 |
| 63 | 0.792 | 0.122 |
| 64 | 0.931 | 0.048 |
| 65 | 0.400 | 0.044 |
| 66 | 0.571 | 0.021 |
| 67 | 0.556 | 0.022 |
| 68 | 0.564 | 0.022 |
| 69 | 0.633 | 0.075 |
| 70 | 0.379 | 0.064 |
| 71 | 0.372 | 0.052 |
| 72 | 0.305 | 0.010 |
| 73 | 0.487 | 0.014 |
| 74 | 0.448 | 0.037 |
| 75 | 0.300 | 0.029 |
| 76 | 0.414 | 0.055 |
| 77 | 0.470 | 0.009 |
| 78 | 0.430 | 0.011 |
| 79 | 0.420 | 0.020 |
| 80 | 0.574 | 0.008 |
| 81 | 0.520 | 0.020 |
| 82 | 0.626 | 0.001 |
| 83 | 0.527 | 0.031 |
| 83 | 0.574 | 0.025 |
| 85 | 0.574 | 0.019 |
| 86 | 0.628 | 0.027 |
| 87 | 0.939 | 0.042 |
| 88 | 0.480 | 0.057 |
| 89 | 0.440 | 0.014 |
| 90 | 0.430 | 0.011 |
| 91 | 0.445 | 0.021 |
| 92 | 0.533 | 0.021 |
| 93 | 0.715 | 0.036 |
| 94 | 0.606 | 0.023 |
| 95 | 0.516 | 0.034 |
| 96 | 0.514 | 0.029 |
| 97 | 0.703 | 0.026 |
| 98 | 0.577 | 0.021 |
| 99 | 0.764 | 0.001 |
| 100 | 0.728 | 0.064 |

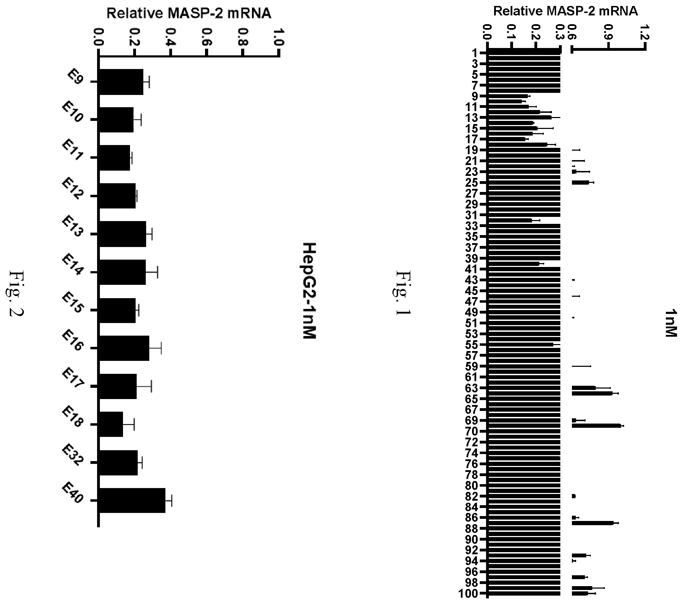

As shown in Fig. 1, the screening of MASP2 dsRNA in HepG2 cells revealed several preferred sequences (No. 9, 10, 11, 12, 13, 14, 15, 16, 17, 18, 32, and 40). dsRNA molecules with good efficacy and sequences simultaneously targeting human and cynomolgus monkey MASP2 were retained as candidate sequences.

### Example 2: Optimization of MASP2-dsRNA: Inhibitory Activity Assay

To further confirm the preferred dsRNA molecules, we performed sequence modification optimization on the above sequences (No. 9, 10, 11, 12, 13, 14, 15, 16, 17, 18, 32, and 40) respectively (Table 4). Fluorination and methoxylation were combined at different positions of the candidate sequences. The overall modification strategy was to replace the antisense strand with methoxylation as much as possible. The sequence design is shown in Table 4. The transfected cells were HepG2 cells, and the synthesis, transfection, quantitative PCR detection steps and PCR primers were the same as in Example 1. Tables 5 and 6 show the average expression levels of target genes relative to the Mock group (the relative mRNA expression level of the Mock group is recorded as 1).

**Table 4: Modified dsRNA sequences**

| Names | Sequences (5'->3') | SEQ ID NO: | Whether an L96 ligand is linked to the 3'-OH at the 3' end |
|---|---|---|---|
| Sense strand of E9 | GmsCmsUmCmCmGmAfCmUfAfCfUmC mCmAmAmCmGmAmsGmsAm | 1 | No |
| Antisense strand of E9 and GE9 | UmsCfsUmCmGmUmUmGmGmAmGmU mAmGfUmCfGmGmAmGmCmsGmsGm | 2 | No |
| Sense strand of E10 | UmsAmsUmCmCmGmAfUmGfGfCfGmC mCmAmCmCmUmAmsAmsUm | 3 | No |
| Antisense strand of E10 and GE10 | AmsUfsUmAmGmGmUmGmGmCmGmC mCmAfUmCfGmGmAmUmAmsAmsGm | 4 | No |
| Sense strand of E11 | CmsAmsCmCmGmUmAfAmCfAfAfGmC mGmCmAmCmCmUmsGmsAm | 5 | No |
| Antisense strand of E11 and GE11 | UmsCfsAmGmGmUmGmCmGmCmUmU mGmUfUmAfCmGmGmUmGmsCmsAm | 6 | No |
| Sense strand of E12 | CmsUmsAmCmCmGmCfCmUfGfCfGmC mCmUmCmUmAmCmsUmsUm | 7 | No |
| Antisense strand of E12 and GE12 | AmsAfsGmUmAmGmAmGmGmCmGmC mAmGfGmCfGmGmUmAmGmsCmsCm | 8 | No |
| Sense strand of E13 | CmsCmsGmUmAmAmCfAmAfGfCfGmC mAmCmCmUmGmCmsUmsAm | 9 | No |
| Antisense strand of E13 and GE13 | UmsAfsGmCmAmGmGmUmGmCmGmC mUmUfGmUfUmAmCmGmGmsUmsGm | 10 | No |
| Sense strand of E14 | CmsCmsUmCmUmGmCfGmAfGfUfAmC mGmAmCmUmUmCmsGmsUm | 11 | No |
| Antisense strand of E14 and GE14 | AmsCfsGmAmAmGmUmCmGmUmAmC mUmCfGmCfAmGmAmGmGmsUmsGm | 12 | No |
| Sense strand of E15 | UmsGmsCmGmAmGmUfAmCfGfAfCmU mUmCmGmUmCmAmsAmsAm | 13 | No |
| Antisense strand of E15 and GE15 | UmsUfsUmGmAmCmGmAmAmGmUmC mGmUfAmCfUmCmGmCmAmsGmsAm | 14 | No |
| Sense strand of E16 | GmsCmsCmUmGmGmAfCmAfUfUfAmC mCmUmUmCmCmGmsCmsUm | 15 | No |
| Antisense strand of E16 and GE16 | AmsGfsCmGmGmAmAmGmGmUmAmA mUmGfUmCfCmAmGmGmCmsUmsGm | 16 | No |
| Sense strand of E17 | GmsAmsAmGmCmCmGfUmUfCfAfCmG mGmGmGmUmUmCmsGmsAm | 17 | No |
| Antisense strand of E17 and GE17 | UmsCfsGmAmAmCmCmCmCmGmUmG mAmAfCmGfGmCmUmUmCmsUmsCm | 18 | No |
| Sense strand of E18 | UmsAmsCmGmUmCmCfUmGfCfAfCmC mGmUmAmAmCmAmsAmsAm | 19 | No |
| Antisense strand of E18 and GE18 | UmsUfsUmGmUmUmAmCmGmGmUmG mCmAfGmGfAmCmGmUmAmsGmsCm | 20 | No |
| Sense strand of E32 | AmsGmsCmUmUmCmUfCmCfAfUfCmU mUmUmUmGmCmGmsAmsAm | 21 | No |
| Antisense strand of E32 and GE32 | UmsUfsCmGmCmAmAmAmAmGmAmU mGmGfAmGfAmAmGmCmUmsGmsUm | 22 | No |
| Sense strand of E40 | UmsGmsCmΛmCmCmGfUmΛfΛfCfΛmΛ mGmCmGmCmAmCmsCmsUm | 23 | No |
| Antisense strand of E40 and GE40 | AmsGfsGmUmGmCmGmCmUmUmGmU mUmAfCmGfGmUmGmCmAmsGmsGm | 24 | No |
| Sense strand of GE9 | GmsCmsUmCmCmGmAfCmUfAfCfUmC mCmAmAmCmGmAmGmAm | 25 | Yes |
| Sense strand of GE10 | UmsAmsUmCmCmGmAfUmGfGfCfGmC mCmAmCmCmUmAmAmUm | 26 | Yes |
| Sense strand of GE11 | CmsAmsCmCmGmUmAfAmCfAfAfGmC mGmCmAmCmCmUmGmAm | 27 | Yes |
| Sense strand of GE12 | CmsUmsAmCmCmGmCfCmUfGfCfGmC mCmUmCmUmAmCmUmUm | 28 | Yes |
| Sense strand of GE13 | CmsCmsGmUmAmAmCfAmAfGfCfGmC mAmCmCmUmGmCmUmAm | 29 | Yes |
| Sense strand of GE14 | CmsCmsUmCmUmGmCfGmAfGfUfAmC mGmAmCmUmUmCmGmUm | 30 | Yes |
| Sense strand of GE15 | UmsGmsCmGmΛmGmUfΛmCfGfΛfCmU mUmCmGmUmCmAmAmAm | 31 | Yes |
| Sense strand of GE16 | GmsCmsCmUmGmGmAfCmAfUfUfAmC mCmUmUmCmCmGmCmUm | 32 | Yes |
| Sense strand of GE17 | GmsAmsAmGmCmCmGfUmUfCfAfCmG mGmGmGmUmUmCmGmAm | 33 | Yes |
| Sense strand of GE18 | UmsAmsCmGmUmCmCfUmGfCfAfCmC mGmUmAmAmCmAmAmAm | 34 | Yes |
| Sense strand of GE32 | AmsGmsCmUmUmCmUfCmCfAfUfCmU mUmUmUmGmCmGmAmAm | 35 | Yes |
| Sense strand of GE40 | UmsGmsCmAmCmCmGfUmAfAfCfAmA mGmCmGmCmAmCmCmUm | 36 | Yes |

**Table 5: Screening of modified dsRNA in HepG2 cells with a 1 nM single dose**

| Names of samples | Relative expression level of MASP2 mRNA (1nM) | Standard error (±SEM) |
|---|---|---|
| E9 | 0.249 | 0.034 |
| E10 | 0.197 | 0.041 |
| E11 | 0.177 | 0.010 |
| E12 | 0.205 | 0.010 |
| E13 | 0.266 | 0.032 |
| E14 | 0.261 | 0.068 |
| E15 | 0.206 | 0.018 |
| E16 | 0.282 | 0.067 |
| E17 | 0.213 | 0.081 |
| E18 | 0.137 | 0.062 |
| E32 | 0.218 | 0.025 |
| E40 | 0.374 | 0.034 |

**Table 6: Screening of modified dsRNA in HepG2 cells with a 0.1 nM single dose**

| Names of samples | Relative expression level of MASP2 mRNA (0.1nM) | Standard error (±SEM) |
|---|---|---|
| E9 | 0.460 | 0.062 |
| E10 | 0.172 | 0.086 |
| E11 | 0.239 | 0.082 |
| E12 | 0.474 | 0.016 |
| E13 | 0.574 | 0.089 |
| E14 | 0.490 | 0.012 |
| E15 | 0.395 | 0.133 |
| E16 | 0.436 | 0.042 |
| E17 | 0.315 | 0.037 |
| E18 | 0.244 | 0.109 |
| E32 | 0.803 | 0.018 |
| E40 | 0.723 | 0.059 |

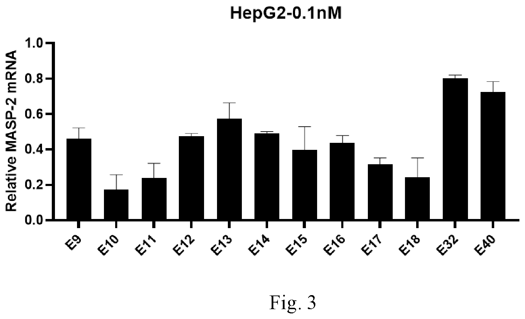

As shown in Figs 2-3, multiple chemically modified dsRNA molecules exhibited high inhibitory activity against target MASP2 mRNA in HepG2 cells. Even at a reduced effective concentration of 0.1 nM, the inhibition rates of dsRNA molecules such as E10, E11, and E18 remained greater than 70%.

### Example 3: In Vivo Efficacy Assay

In this experiment, 6-8 week old SPF-grade humanized MASP2 mice (Shanghai Model Organisms Center, Inc.) were randomly divided into 5 groups: the saline group, and administration groups (including E10, E11, E17 and E18 groups), 10 animals per group (N=10, 5 females and 5 males). The medicament was administered subcutaneously by a single injection at 3 mpk (i.e., mg/kg) (L96-modified siRNA). Blood samples were collected on D-3 (3 days before administration), D3, D7, and D12 (D0 is the day of administration). Serum was collected by phlebotomy from behind the eyeball, and the MASP2 protein level was detected by ELISA.

Preliminary in vivo data showed that on day 12, the inhibition rate of MASP2 protein in the serum of each treatment group was >50%, or even higher.

The above description is merely preferred embodiments and is intended as examples only, without limiting the combination of features necessary for implementing this application. The provided headings are not intended to limit the various embodiments of this application. Terms such as "comprise," "contain," and "include" are not intended to be restrictive. In addition, unless otherwise stated, the plural form is included when there is no numeral modifier, and the word "or" means "and/or". Unless otherwise defined herein, all technical and scientific terms used herein have the same meaning as commonly understood by those skilled in the art. All disclosures and patents mentioned in this application are incorporated herein by reference. Various modifications and variations of the methods and compositions described herein will be apparent to those skilled in the art without departing from the scope and spirit of this application. Although this application has been described through specific preferred embodiments, it should be understood that the claimed application should not be unduly limited to these specific embodiments. In fact, the various variations of the described modes for implementing this application that are obvious to those skilled in the art are intended to be included within the scope of the appended claims.

## Claims

1. An engineered dsRNA molecule for inhibiting the expression of complement MASP2 gene, wherein the molecule comprises a sense strand and an antisense strand complementary to each other to form a double-stranded region, the sense strand and/or the antisense strand comprises or consists of 15-25 nucleotides, the antisense strand is complementary to at least 15, 16, 17, 18, 19, 20 or 21 consecutive nucleotides of the nucleic acid sequence set forth in SEQ ID NO: 37, 39, 41, 43, 45, 47, 49, 51, 53, 55, 71 or 77, and the length of the double-stranded region is 15-25 bp.

2. At least one nucleotide in the dsRNA molecule is modified, wherein at least one nucleotide has a modification, and the modification is any one or more selected from the group consisting of: locked nucleic acid modification, acyclic or unlocked nucleic acid modification, 2'-methoxyethyl modification, 2'-O-methyl modification, 2'-O-allyl modification, 2'-C-allyl modification, 2'-fluoro modification, 2'-deoxy modification, phosphorothioate backbone modification, DNA modification, and ligand modification.

3. The dsRNA molecule according to claim 1 or 2, wherein, with respect to the naked sequence thereof:
the sense strand of the dsRNA molecule comprises or consists of the nucleic acid sequence set forth in SEQ ID NO: 37, and the antisense strand comprises or consists of the nucleic acid sequence set forth in SEQ ID NO: 38;
the sense strand of the dsRNA molecule comprises or consists of the nucleic acid sequence set forth in SEQ ID NO: 39, and the antisense strand comprises or consists of the nucleic acid sequence set forth in SEQ ID NO: 40;
the sense strand of the dsRNA molecule comprises or consists of the nucleic acid sequence set forth in SEQ ID NO: 41, and the antisense strand comprises or consists of the nucleic acid sequence set forth in SEQ ID NO: 42;
the sense strand of the dsRNA molecule comprises or consists of the nucleic acid sequence set forth in SEQ ID NO: 43, and the antisense strand comprises or consists of the nucleic acid sequence set forth in SEQ ID NO: 44;
the sense strand of the dsRNA molecule comprises or consists of the nucleic acid sequence set forth in SEQ ID NO: 45, and the antisense strand comprises or consists of the nucleic acid sequence set forth in SEQ ID NO: 46;
the sense strand of the dsRNA molecule comprises or consists of the nucleic acid sequence set forth in SEQ ID NO: 47, and the antisense strand comprises or consists of the nucleic acid sequence set forth in SEQ ID NO: 48;
the sense strand of the dsRNA molecule comprises or consists of the nucleic acid sequence set forth in SEQ ID NO: 49, and the antisense strand comprises or consists of the nucleic acid sequence set forth in SEQ ID NO: 50;
the sense strand of the dsRNA molecule comprises or consists of the nucleic acid sequence set forth in SEQ ID NO: 51, and the antisense strand comprises or consists of the nucleic acid sequence set forth in SEQ ID NO: 52;
the sense strand of the dsRNA molecule comprises or consists of the nucleic acid sequence set forth in SEQ ID NO: 53, and the antisense strand comprises or consists of the nucleic acid sequence set forth in SEQ ID NO: 54;
the sense strand of the dsRNA molecule comprises or consists of the nucleic acid sequence set forth in SEQ ID NO: 55, and the antisense strand comprises or consists of the nucleic acid sequence set forth in SEQ ID NO: 56;
the sense strand of the dsRNA molecule comprises or consists of the nucleic acid sequence set forth in SEQ ID NO: 71, and the antisense strand comprises or consists of the nucleic acid sequence set forth in SEQ ID NO: 72; or
the sense strand of the dsRNA molecule comprises or consists of the nucleic acid sequence set forth in SEQ ID NO: 77, and the antisense strand comprises or consists of the nucleic acid sequence set forth in SEQ ID NO: 78.

4. The dsRNA molecule according to any one of claims 1-3, wherein the modification pattern of the dsRNA molecule comprises:
(1) a sense strand: 19-23 nt in length, such as 19, 20, 21, 22, or 23 nt; consisting of alternating 2'-O-methyl and 2'-fluoro modified regions, wherein the number of the consecutive nucleotides in each modified region is any one of 1, 2, 3, 4, 5, 6, 7, 8, 9, 10, 11, or 12 nucleotides; and
(2) an antisense strand: 19-25 nt in length, such as 19, 20, 21, 22, 23nt, 24n or 25nt; consisting of alternating 2'-O-methyl modified region, 2'-fluoro modified region, unmodified region and/or DNA region, wherein the number of the consecutive nucleotides in each modified region is any one of 1, 2, 3, 4, 5, 6, 7, 8, 9, 10, 11 or 12 nucleotides; the modification patterns of the first modified region from the 5' end and the 3' end are the same;
furthermore, in the sense strand and antisense strand, the consecutive nucleotide regions at positions 1-2, 1-3, 1-4, 1-5, 1-6, or 1-7 starting from the 5' end, and optionally the consecutive nucleotide regions at positions 1-2, 1-3, 1-4, 1-5, 1-6, or 1-7 starting from the 3' end, are all linked by a phosphorothioate backbone; preferably, the consecutive nucleotide regions at positions 1-3 starting from the 5' end, and optionally the consecutive nucleotide regions at positions 1-3 starting from the 3' end, are all linked by a phosphorothioate backbone.

5. The dsRNA molecule according to any one of claims 1-4, wherein the ligand in the ligand modification is any one or more selected from the group consisting of: cholesterol, biotin, vitamin, galactose derivative or analog, lactose derivative or analog, N-acetylgalactosamine derivative or analog, and N-acetylglucosamine derivative or analog.

6. The dsRNA molecule according to claim 5, wherein the ligand is linked to the 3' terminal nucleotide of the sense strand and/or antisense strand; the conjugation is linked to a base or a sugar ring; preferably, the ligand is linked to a sugar ring; more preferably, the ligand is linked to the 3' position of the sugar ring.

7. The dsRNA molecule according to claim 6, wherein the ligand is one or more GalNAc derivatives linked by a divalent or trivalent branched structure;
preferably, the GalNAc derivative comprises the following structure: 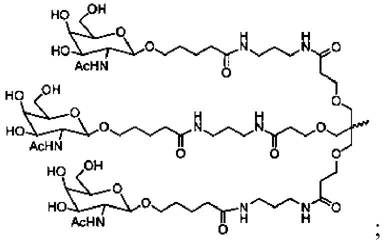
more preferably, the ligand is L96 with a structure shown in the following Formula I:

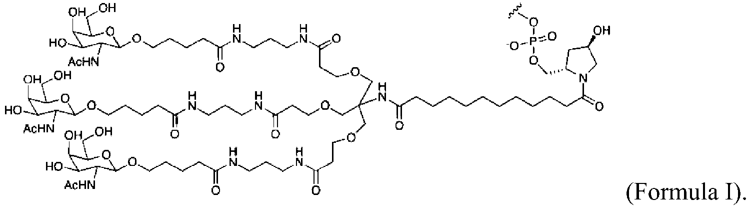

8. The dsRNA molecule according to any one of claims 1-7, wherein it comprises a modification motif of any one selected from the group consisting of:
(1) a sense strand:
NmsNmsNmNmNmNmNfNmNfNfNfNmNmNmNmNmNmNmNmsNmsNm, and
an antisense strand:
NmsNfsNmNmNmNmNmNmNmNmNmNmNmNfNmNfNmNmNmNmNmsNmsNm;
(2) a sense strand:
NmsNmsNmNmNmNmNfNmNfNfNfNmNmNmNmNmNmNmNmNmNm, and
an antisense strand:
NmsNfsNmNmNmNmNmNmNmNmNmNmNmNfNmNfNmNmNmNmNmsNmsNm;
wherein Nm represents a ribonucleotide modified with 2'-O-methyl; Nf represents a ribonucleotide modified with 2'-fluoro; and (s) indicates that the two adjacent nucleotides are linked by a phosphorothioate backbone.

9. The double-stranded RNAi molecule according to any one of claims 1-8, wherein it comprises one or more selected from the group consisting of:
(1) a sense strand consisting of a nucleotide sequence having at least 90%, preferably 95%, 96%, 97%, 98%, 99%, or 100% identity with the nucleotide sequence and corresponding modifications set forth in SEQ ID NO: 1, and an antisense strand consisting of a nucleotide sequence having at least 90%, preferably 95%, 96%, 97%, 98%, 99%, or 100% identity with the nucleotide sequence and corresponding modifications set forth in SEQ ID NO: 2;
(2) a sense strand consisting of a nucleotide sequence having at least 90%, preferably 95%, 96%, 97%, 98%, 99%, or 100% identity with the nucleotide sequence and corresponding modifications set forth in SEQ ID NO: 3, and an antisense strand consisting of a nucleotide sequence having at least 90%, preferably 95%, 96%, 97%, 98%, 99%, or 100% identity with the nucleotide sequence and corresponding modifications set forth in SEQ ID NO: 4;
(3) a sense strand consisting of a nucleotide sequence having at least 90%, preferably 95%, 96%, 97%, 98%, 99%, or 100% identity with the nucleotide sequence and corresponding modifications set forth in SEQ ID NO: 5, and an antisense strand consisting of a nucleotide sequence having at least 90%, preferably 95%, 96%, 97%, 98%, 99%, or 100% identity with the nucleotide sequence and corresponding modifications set forth in SEQ ID NO: 6;
(4) a sense strand consisting of a nucleotide sequence having at least 90%, preferably 95%, 96%, 97%, 98%, 99%, or 100% identity with the nucleotide sequence and corresponding modifications set forth in SEQ ID NO: 7, and an antisense strand consisting of a nucleotide sequence having at least 90%, preferably 95%, 96%, 97%, 98%, 99%, or 100% identity with the nucleotide sequence and corresponding modifications set forth in SEQ ID NO: 8;
(5) a sense strand consisting of a nucleotide sequence having at least 90%, preferably 95%, 96%, 97%, 98%, 99%, or 100% identity with the nucleotide sequence and corresponding modifications set forth in SEQ ID NO: 9, and an antisense strand consisting of a nucleotide sequence having at least 90%, preferably 95%, 96%, 97%, 98%, 99%, or 100% identity with the nucleotide sequence and corresponding modifications set forth in SEQ ID NO: 10;
(6) a sense strand consisting of a nucleotide sequence having at least 90%, preferably 95%, 96%, 97%, 98%, 99%, or 100% identity with the nucleotide sequence and corresponding modifications set forth in SEQ ID NO: 11, and an antisense strand consisting of a nucleotide sequence having at least 90%, preferably 95%, 96%, 97%, 98%, 99%, or 100% identity with the nucleotide sequence and corresponding modifications set forth in SEQ ID NO: 12;
(7) a sense strand consisting of a nucleotide sequence having at least 90%, preferably 95%, 96%, 97%, 98%, 99%, or 100% identity with the nucleotide sequence and corresponding modifications set forth in SEQ ID NO: 13, and an antisense strand consisting of a nucleotide sequence having at least 90%, preferably 95%, 96%, 97%, 98%, 99%, or 100% identity with the nucleotide sequence and corresponding modifications set forth in SEQ ID NO: 14;
(8) a sense strand consisting of a nucleotide sequence having at least 90%, preferably 95%, 96%, 97%, 98%, 99%, or 100% identity with the nucleotide sequence and corresponding modifications set forth in SEQ ID NO: 15, and an antisense strand consisting of a nucleotide sequence having at least 90%, preferably 95%, 96%, 97%, 98%, 99%, or 100% identity with the nucleotide sequence and corresponding modifications set forth in SEQ ID NO: 16;
(9) a sense strand consisting of a nucleotide sequence having at least 90%, preferably 95%, 96%, 97%, 98%, 99%, or 100% identity with the nucleotide sequence and corresponding modifications set forth in SEQ ID NO: 17, and an antisense strand consisting of a nucleotide sequence having at least 90%, preferably 95%, 96%, 97%, 98%, 99%, or 100% identity with the nucleotide sequence and corresponding modifications set forth in SEQ ID NO: 18;
(10) a sense strand consisting of a nucleotide sequence having at least 90%, preferably 95%, 96%, 97%, 98%, 99%, or 100% identity with the nucleotide sequence and corresponding modifications set forth in SEQ ID NO: 19, and an antisense strand consisting of a nucleotide sequence having at least 90%, preferably 95%, 96%, 97%, 98%, 99%, or 100% identity with the nucleotide sequence and corresponding modifications set forth in SEQ ID NO: 20;
(11) a sense strand consisting of a nucleotide sequence having at least 90%, preferably 95%, 96%, 97%, 98%, 99%, or 100% identity with the nucleotide sequence and corresponding modifications set forth in SEQ ID NO: 21, and an antisense strand consisting of a nucleotide sequence having at least 90%, preferably 95%, 96%, 97%, 98%, 99%, or 100% identity with the nucleotide sequence and corresponding modifications set forth in SEQ ID NO: 22;
(12) a sense strand consisting of a nucleotide sequence having at least 90%, preferably 95%, 96%, 97%, 98%, 99%, or 100% identity with the nucleotide sequence and corresponding modifications set forth in SEQ ID NO: 23, and an antisense strand consisting of a nucleotide sequence having at least 90%, preferably 95%, 96%, 97%, 98%, 99%, or 100% identity with the nucleotide sequence and corresponding modifications set forth in SEQ ID NO: 24;
(13) a sense strand consisting of a nucleotide sequence having at least 90%, preferably 95%, 96%, 97%, 98%, 99%, or 100% identity with the nucleotide sequence and corresponding modifications set forth in SEQ ID NO: 25, and an antisense strand consisting of a nucleotide sequence having at least 90%, preferably 95%, 96%, 97%, 98%, 99%, or 100% identity with the nucleotide sequence and corresponding modifications set forth in SEQ ID NO: 2;
(14) a sense strand consisting of a nucleotide sequence having at least 90%, preferably 95%, 96%, 97%, 98%, 99%, or 100% identity with the nucleotide sequence and corresponding modifications set forth in SEQ ID NO: 26, and an antisense strand consisting of a nucleotide sequence having at least 90%, preferably 95%, 96%, 97%, 98%, 99%, or 100% identity with the nucleotide sequence and corresponding modifications set forth in SEQ ID NO: 4;
(15) a sense strand consisting of a nucleotide sequence having at least 90%, preferably 95%, 96%, 97%, 98%, 99%, or 100% identity with the nucleotide sequence and corresponding modifications set forth in SEQ ID NO: 27, and an antisense strand consisting of a nucleotide sequence having at least 90%, preferably 95%, 96%, 97%, 98%, 99%, or 100% identity with the nucleotide sequence and corresponding modifications set forth in SEQ ID NO: 6;
(16) a sense strand consisting of a nucleotide sequence having at least 90%, preferably 95%, 96%, 97%, 98%, 99%, or 100% identity with the nucleotide sequence and corresponding modifications set forth in SEQ ID NO: 28, and an antisense strand consisting of a nucleotide sequence having at least 90%, preferably 95%, 96%, 97%, 98%, 99%, or 100% identity with the nucleotide sequence and corresponding modifications set forth in SEQ ID NO: 8;
(17) a sense strand consisting of a nucleotide sequence having at least 90%, preferably 95%, 96%, 97%, 98%, 99%, or 100% identity with the nucleotide sequence and corresponding modifications set forth in SEQ ID NO: 29, and an antisense strand consisting of a nucleotide sequence having at least 90%, preferably 95%, 96%, 97%, 98%, 99%, or 100% identity with the nucleotide sequence and corresponding modifications set forth in SEQ ID NO: 10;
(18) a sense strand consisting of a nucleotide sequence having at least 90%, preferably 95%, 96%, 97%, 98%, 99%, or 100% identity with the nucleotide sequence and corresponding modifications set forth in SEQ ID NO: 30, and an antisense strand consisting of a nucleotide sequence having at least 90%, preferably 95%, 96%, 97%, 98%, 99%, or 100% identity with the nucleotide sequence and corresponding modifications set forth in SEQ ID NO: 12;
(19) a sense strand consisting of a nucleotide sequence having at least 90%, preferably 95%, 96%, 97%, 98%, 99%, or 100% identity with the nucleotide sequence and corresponding modifications set forth in SEQ ID NO: 31, and an antisense strand consisting of a nucleotide sequence having at least 90%, preferably 95%, 96%, 97%, 98%, 99%, or 100% identity with the nucleotide sequence and corresponding modifications set forth in SEQ ID NO: 14;
(20) a sense strand consisting of a nucleotide sequence having at least 90%, preferably 95%, 96%, 97%, 98%, 99%, or 100% identity with the nucleotide sequence and corresponding modifications set forth in SEQ ID NO: 32, and an antisense strand consisting of a nucleotide sequence having at least 90%, preferably 95%, 96%, 97%, 98%, 99%, or 100% identity with the nucleotide sequence and corresponding modifications set forth in SEQ ID NO: 16;
(21) a sense strand consisting of a nucleotide sequence having at least 90%, preferably 95%, 96%, 97%, 98%, 99%, or 100% identity with the nucleotide sequence and corresponding modifications set forth in SEQ ID NO: 33, and an antisense strand consisting of a nucleotide sequence having at least 90%, preferably 95%, 96%, 97%, 98%, 99%, or 100% identity with the nucleotide sequence and corresponding modifications set forth in SEQ ID NO: 18;
(22) a sense strand consisting of a nucleotide sequence having at least 90%, preferably 95%, 96%, 97%, 98%, 99%, or 100% identity with the nucleotide sequence and corresponding modifications set forth in SEQ ID NO: 34, and an antisense strand consisting of a nucleotide sequence having at least 90%, preferably 95%, 96%, 97%, 98%, 99%, or 100% identity with the nucleotide sequence and corresponding modifications set forth in SEQ ID NO: 20;
(23) a sense strand consisting of a nucleotide sequence having at least 90%, preferably 95%, 96%, 97%, 98%, 99%, or 100% identity with the nucleotide sequence and corresponding modifications set forth in SEQ ID NO: 35, and an antisense strand consisting of a nucleotide sequence having at least 90%, preferably 95%, 96%, 97%, 98%, 99%, or 100% identity with the nucleotide sequence and corresponding modifications set forth in SEQ ID NO: 22; and
(24) a sense strand consisting of a nucleotide sequence having at least 90%, preferably 95%, 96%, 97%, 98%, 99%, or 100% identity with the nucleotide sequence and corresponding modifications set forth in SEQ ID NO: 36, and an antisense strand consisting of a nucleotide sequence having at least 90%, preferably 95%, 96%, 97%, 98%, 99%, or 100% identity with the nucleotide sequence and corresponding modifications set forth in SEQ ID NO: 24.

10. The double-stranded RNAi molecule according to claim 9, wherein its nucleotide sequences comprise one or more selected from the the group consisting of:
(1) a sense strand with a nucleotide sequence consisting of SEQ ID NO: 1 and 0-5 additional nucleotides at the 5' and/or 3' ends, and an antisense strand with the nucleotide sequence consisting of SEQ ID NO: 2 and 0-5 additional nucleotides at the 5' and/or 3' ends;
(2) a sense strand with a nucleotide sequence consisting of SEQ ID NO: 3 and 0-5 additional nucleotides at the 5' and/or 3' ends, and an antisense strand with the nucleotide sequence consisting of SEQ ID NO: 4 and 0-5 additional nucleotides at the 5' and/or 3' ends;
(3) a sense strand with a nucleotide sequence consisting of SEQ ID NO: 5 and 0-5 additional nucleotides at the 5' and/or 3' ends, and an antisense strand with the nucleotide sequence consisting of SEQ ID NO: 6 and 0-5 additional nucleotides at the 5' and/or 3' ends;
(4) a sense strand with a nucleotide sequence consisting of SEQ ID NO: 7 and 0-5 additional nucleotides at the 5' and/or 3' ends, and an antisense strand with the nucleotide sequence consisting of SEQ ID NO: 8 and 0-5 additional nucleotides at the 5' and/or 3' ends;
(5) a sense strand with a nucleotide sequence consisting of SEQ ID NO: 9 and 0-5 additional nucleotides at the 5' and/or 3' ends, and an antisense strand with the nucleotide sequence consisting of SEQ ID NO: 10 and 0-5 additional nucleotides at the 5' and/or 3' ends;
(6) a sense strand with a nucleotide sequence consisting of SEQ ID NO: 11 and 0-5 additional nucleotides at the 5' and/or 3' ends, and an antisense strand with the nucleotide sequence consisting of SEQ ID NO: 12 and 0-5 additional nucleotides at the 5' and/or 3' ends;
(7) a sense strand with a nucleotide sequence consisting of SEQ ID NO: 13 and 0-5 additional nucleotides at the 5' and/or 3' ends, and an antisense strand with the nucleotide sequence consisting of SEQ ID NO: 14 and 0-5 additional nucleotides at the 5' and/or 3' ends;
(8) a sense strand with a nucleotide sequence consisting of SEQ ID NO: 15 and 0-5 additional nucleotides at the 5' and/or 3' ends, and an antisense strand with the nucleotide sequence consisting of SEQ ID NO: 16 and 0-5 additional nucleotides at the 5' and/or 3' ends;
(9) a sense strand with a nucleotide sequence consisting of SEQ ID NO: 17 and 0-5 additional nucleotides at the 5' and/or 3' ends, and an antisense strand with the nucleotide sequence consisting of SEQ ID NO: 18 and 0-5 additional nucleotides at the 5' and/or 3' ends;
(10) a sense strand with a nucleotide sequence consisting of SEQ ID NO: 19 and 0-5 additional nucleotides at the 5' and/or 3' ends, and an antisense strand with the nucleotide sequence consisting of SEQ ID NO: 20 and 0-5 additional nucleotides at the 5' and/or 3' ends;
(11) a sense strand with a nucleotide sequence consisting of SEQ ID NO: 21 and 0-5 additional nucleotides at the 5' and/or 3' ends, and an antisense strand with the nucleotide sequence consisting of SEQ ID NO: 22 and 0-5 additional nucleotides at the 5' and/or 3' ends;
(12) a sense strand with a nucleotide sequence consisting of SEQ ID NO: 23 and 0-5 additional nucleotides at the 5' and/or 3' ends, and an antisense strand with the nucleotide sequence consisting of SEQ ID NO: 24 and 0-5 additional nucleotides at the 5' and/or 3' ends;
(13) a sense strand with a nucleotide sequence consisting of SEQ ID NO: 25 and 0-5 additional nucleotides at the 5' and/or 3' ends, and an antisense strand with the nucleotide sequence consisting of SEQ ID NO: 2 and 0-5 additional nucleotides at the 5' and/or 3' ends;
(14) a sense strand with a nucleotide sequence consisting of SEQ ID NO: 26 and 0-5 additional nucleotides at the 5' and/or 3' ends, and an antisense strand with the nucleotide sequence consisting of SEQ ID NO: 4 and 0-5 additional nucleotides at the 5' and/or 3' ends;
(15) a sense strand with a nucleotide sequence consisting of SEQ ID NO: 27 and 0-5 additional nucleotides at the 5' and/or 3' ends, and an antisense strand with the nucleotide sequence consisting of SEQ ID NO: 6 and 0-5 additional nucleotides at the 5' and/or 3' ends;
(16) a sense strand with a nucleotide sequence consisting of SEQ ID NO: 28 and 0-5 additional nucleotides at the 5' and/or 3' ends, and an antisense strand with the nucleotide sequence consisting of SEQ ID NO: 8 and 0-5 additional nucleotides at the 5' and/or 3' ends;
(17) a sense strand with a nucleotide sequence consisting of SEQ ID NO: 29 and 0-5 additional nucleotides at the 5' and/or 3' ends, and an antisense strand with the nucleotide sequence consisting of SEQ ID NO: 10 and 0-5 additional nucleotides at the 5' and/or 3' ends;
(18) a sense strand with a nucleotide sequence consisting of SEQ ID NO: 30 and 0-5 additional nucleotides at the 5' and/or 3' ends, and an antisense strand with the nucleotide sequence consisting of SEQ ID NO: 12 and 0-5 additional nucleotides at the 5' and/or 3' ends;
(19) a sense strand with a nucleotide sequence consisting of SEQ ID NO: 31 and 0-5 additional nucleotides at the 5' and/or 3' ends, and an antisense strand with the nucleotide sequence consisting of SEQ ID NO: 14 and 0-5 additional nucleotides at the 5' and/or 3' ends;
(20) a sense strand with a nucleotide sequence consisting of SEQ ID NO: 32 and 0-5 additional nucleotides at the 5' and/or 3' ends, and an antisense strand with the nucleotide sequence consisting of SEQ ID NO: 16 and 0-5 additional nucleotides at the 5' and/or 3' ends;
(21) a sense strand with a nucleotide sequence consisting of SEQ ID NO: 33 and 0-5 additional nucleotides at the 5' and/or 3' ends, and an antisense strand with the nucleotide sequence consisting of SEQ ID NO: 18 and 0-5 additional nucleotides at the 5' and/or 3' ends;
(22) a sense strand with a nucleotide sequence consisting of SEQ ID NO: 34 and 0-5 additional nucleotides at the 5' and/or 3' ends, and an antisense strand with the nucleotide sequence consisting of SEQ ID NO: 20 and 0-5 additional nucleotides at the 5' and/or 3' ends;
(23) a sense strand with a nucleotide sequence consisting of SEQ ID NO: 35 and 0-5 additional nucleotides at the 5' and/or 3' ends, and an antisense strand with the nucleotide sequence consisting of SEQ ID NO: 22 and 0-5 additional nucleotides at the 5' and/or 3' ends; and
(24) a sense strand with a nucleotide sequence consisting of SEQ ID NO: 36 and 0-5 additional nucleotides at the 5' and/or 3' ends, and an antisense strand with the nucleotide sequence consisting of SEQ ID NO: 24 and 0-5 additional nucleotides at the 5' and/or 3' ends.

11. The double-stranded RNAi molecule according to any one of claims 1-10, wherein it comprises any one or more selected from the group consisting of:
(1) a sense strand with the nucleotide sequence set forth in SEQ ID NO: 25, and an antisense strand with the nucleotide sequence set forth in SEQ ID NO: 2;
(2) a sense strand with the nucleotide sequence set forth in SEQ ID NO: 26, and an antisense strand with the nucleotide sequence set forth in SEQ ID NO: 4;
(3) a sense strand with the nucleotide sequence set forth in SEQ ID NO: 27, and an antisense strand with the nucleotide sequence set forth in SEQ ID NO: 6;
(4) a sense strand with the nucleotide sequence set forth in SEQ ID NO: 28, and an antisense strand with the nucleotide sequence set forth in SEQ ID NO: 8;
(5) a sense strand with the nucleotide sequence set forth in SEQ ID NO: 29, and an antisense strand with the nucleotide sequence set forth in SEQ ID NO: 10;
(6) a sense strand with the nucleotide sequence set forth in SEQ ID NO: 30, and an antisense strand with the nucleotide sequence set forth in SEQ ID NO: 12;
(7) a sense strand with the nucleotide sequence set forth in SEQ ID NO: 31, and an antisense strand with the nucleotide sequence set forth in SEQ ID NO: 14;
(8) a sense strand with the nucleotide sequence set forth in SEQ ID NO: 32, and an antisense strand with the nucleotide sequence set forth in SEQ ID NO: 16;
(9) a sense strand with the nucleotide sequence set forth in SEQ ID NO: 33, and an antisense strand with the nucleotide sequence set forth in SEQ ID NO: 18;
(10) a sense strand with the nucleotide sequence set forth in SEQ ID NO: 34, and an antisense strand with the nucleotide sequence set forth in SEQ ID NO: 20;
(11) a sense strand with the nucleotide sequence set forth in SEQ ID NO: 35, and an antisense strand with the nucleotide sequence set forth in SEQ ID NO: 22; and
(12) a sense strand with the nucleotide sequence set forth in SEQ ID NO: 36, and an antisense strand with the nucleotide sequence set forth in SEQ ID NO: 24;
wherein the 3'-OH at the 3' end of the sense strand is linked with an L96 ligand as shown in the following Formula I:

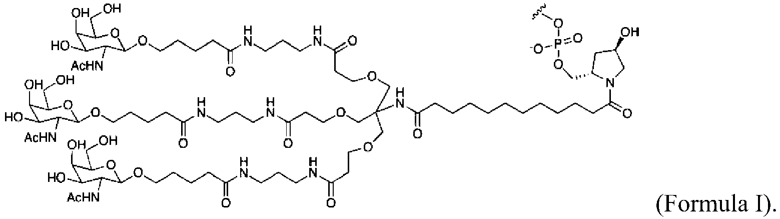

12. A biomaterial, wherein it is any one selected from the group consisting of:
(A) a reagent or kit comprising the dsRNA molecule according to any one of claims 1-11 or a DNA molecule encoding the dsRNA molecule; and
(B) a pharmaceutical composition comprising the dsRNA molecule according to any one of claims 1-11 and other pharmaceutically acceptable components.

13. Use of a dsRNA, wherein it is any one selected from the group consisting of:
(I) use of the dsRNA molecule according to any one of claims 1-11 or the biomaterial according to claim 12 in inhibiting complement MASP2 gene expression or in the preparation of a product for inhibiting complement MASP2 gene expression;
(II) use of the dsRNA molecule according to any one of claims 1-11 or the biomaterial according to claim 12 in the preparation of a product for reducing MASP2 proteins in serum;
(III) use of the dsRNA molecule according to any one of claims 1-11 or the biomaterial according to claim 12 in the prevention and/or treatment of a disease mediated by the complement MASP2 gene, or in the preparation of a product for the prevention and/or treatment of a disease mediated by the complement MASP2 gene;
(IV) use of the dsRNA molecule according to any one of claims 1-11 or the biomaterial according to claim 12 in alleviating symptoms of a disease mediated by the complement MASP2 gene, or in the preparation of a product for alleviating symptoms of a disease mediated by the complement MASP2 gene;
the disease mediated by the complement MASP2 gene comprises: ophthalmic disease, hematological disease, cardiovascular disease, autoimmune disease, kidney disease, neurological disease, or oncological disease; wherein the ophthalmic disease comprises dry/wet age-related macular degeneration (AMD), geographic atrophy (GA), etc.; the neurological disease comprises: Alzheimer's disease (AD), myasthenia gravis (gMG), etc.; the kidney disease comprises: typical hemolytic uremic syndrome (aHUS), C3 glomerulonephropathy (C3G), and IgA nephropathy; the hematological disease comprises: paroxysmal nocturnal hemoglobinuria (PNH), thrombotic microangiopathy (TMAs); the autoimmune disease comprises: rheumatoid arthritis, lupus erythematosus, etc.; and the oncological disease comprises: complement-related liver cancer or lung cancer.
